# EUROPEAN PATENT SPECIFICATION

(11) **EP 3 723 791 B1**
(45) Date of publication and mention of the grant of the patent: **21.02.2024**
(21) Application number: 18829780.8
(22) Date of filing: 14.12.2018
(51) Int. Cl.: A61K 39/00, C07K 16/28, C07K 16/46, A61K 47/68, A61P 37/06, C07K 16/00

(54) **RECOMBINANT IGG FC MULTIMERS FOR THE TREATMENT OF NEUROMYELITIS OPTICA**
REKOMBINANTE IGG-FC-MULTIMERE ZUR BEHANDLUNG VON NEUROMYELITIS OPTICA
MULTIMÈRES DE FC D'IGG DE RECOMBINAISON POUR LE TRAITEMENT DE LA NEUROMYÉLITE OPTIQUE

(30) Priority: 14.12.2017 US 201762598592 P; 20.04.2018 EP 18168448
(43) Date of publication of application: 21.10.2020
(73) Proprietor: CSL Behring Lengnau AG, 2543 Lengnau BE (CH); The Regents of the University of California, Oakland, CA 94607 (US)
(72) Inventor: SPIRIG, Rolf, 3007 Bern (CH); BAZ MORELLI, Adriana, Carlton, VIC 3053 (AU); VERKMAN, Alan, San Francisco, CA 94127 (US); TRADTRANTIP, Lukmanee, San Francisco, CA 94127 (US)
(74) Representative: CSL Global IP
(86) International application number: PCT/EP2018/084894
(87) International publication number: WO 2019/115745

(56) References cited:
- WO-A1-2015/132364
- WO-A1-2015/132365
- WO-A1-2016/139365
- WO-A1-2017/005767
- WO-A1-2017/129737
- TRADTRANTIP LUKMANEE ET AL: "Recombinant IgG1 Fc hexamers block cytotoxicity and pathological changes in experimental in vitro and rat models of neuromyelitis optica.", NEUROPHARMACOLOGY, vol. 133, 8 February 2018 (2018-02-08), pages 345-353, XP002785016, ISSN: 1873-7064
- SPIRIG ROLF ET AL: "rIgG1 Fc Hexamer Inhibits Antibody-Mediated Autoimmune Disease via Effects on Complement and Fc[gamma]Rs.", JOURNAL OF IMMUNOLOGY (BALTIMORE, MD. : 1950) 12 MAR 2018, vol. 200, no. 8, 12 March 2018 (2018-03-12), pages 2542-2553, XP002785017, ISSN: 1550-6606
- SØRENSEN V ET AL: "Effect of the IgM and IgA secretory tailpieces on polymerization and secretion of IgM and IgG.", JOURNAL OF IMMUNOLOGY (BALTIMORE, MD. : 1950) 15 APR 1996, vol. 156, no. 8, 15 April 1996 (1996-04-15), pages 2858-2865, XP002785018, ISSN: 0022-1767

## Description

### FIELD OF THE INVENTION

This disclosure provides hexameric recombinant IgG Fc multimers for use in the treatment of neuromyelitis optica (NMO).

### BACKGROUND

Plasma-derived immunoglobulin G (IgG) is used in the clinics to treat primary and secondary immunodeficiency. In this case, IgG is administered either intravenously (IVIG) or subcutaneously (SCIG). Both are prepared from large plasma pools of more than 10,000 donors, ensuring a diverse antibody repertoire.

The administration of high doses of IVIG (1-2 g/kg/dose) has been increasingly used for the treatment of patients with chronic or acute autoimmune and inflammatory diseases such as immune thrombo cytopenia (ITP), Guillain-Barré syndrome, Kawasaki disease, chronic inflammatory demyelinating polyneuropathy (CIDP), myasthenia gravis (MG), and several other rare diseases. Additionally, off-label uses of IVIG for several other indications are currently under exploration such as, for example, for the treatment of rheumatoid arthritis (RA).

Numerous mechanisms of action have been proposed for the anti-inflammatory effect of high-dose IVIG. These include blockage of Fcy receptors (FcyRs), saturation of neonatal FcR (FcRn) to enhance autoantibody clearance, up-regulation of inhibitory FcyRIIB (CD32B), scavenging of complement protein fragments and inhibition of complement fragment deposition, anti-idiotypic antibodies (Abs) in IVIG, binding or neutralization of immune mediators (e.g. cytokines), or modulation of immune cells (e.g. induction of regulatory T cells, B cells or tolerogenic dendritic cells).

There is a need for effective and safe therapy for neuromyelitis optica spectrum disorders (herein called NMO), an autoimmune demyelinating disease of the central nervous system characterized by astrocyte injury, inflammation and demyelination (Hengstman et al., 2007. Mult. Scler. 13, 679-682; Misu et al., 2007 Brain 130, 1224-1234; Papadopoulos and Verkman, 2012, Lancet Neurol. 11, 535-544). Current therapeutics include immunosuppressants, plasma exchange and B cell depletion, and several drugs are under evaluation or in pre-clinical development targeting various NMO pathogenesis mechanisms such as complement, IL-6 receptors and NMO autoantibody interactions (Araki et al., 2014, Neurology 82, 1302-1306; Cree et al., 2005, Neurology 64, 1270-1272; Greenberg et al., 2012, Mult. Scler. 18, 1022-1026; Kageyama et al., 2013, J. Neurol. 260, 627-634; Papadopoulos et al., 2014, Nat. Rev. Neurol. 10, 493-506; Verkman et al., 2013, Brain Pathol. 23, 84-695). Most NMO patients are seropositive for IgG1 autoantibodies against aquaporin-4 (AQP4) (called AQP4-IgG or NMO-IgG), a water channel expressed on astrocytes in which AQP4-IgG binding to AQP4 causes primary injury to astrocytes by complement and cellular effector mechanisms, producing inflammation and oligodendrocyte injury (Asgari et al., 2013, J. Neuroimmunol. 254, 76-82; Graber et al., 2008, J. Neuroinflam. 5, 22; Jarius et al., 2014; Jarius and Wildemann, 2010, Nat. Rev. Neurol. 6, 383-392; Lennon et al., 2005, J. Exp. Med. 202, 473-477; Lucchinetti et al., 2002, Brain 125, 1450-1461; Parratt and Prineas, 2010, Mult. Scler. 16, 1156-1172).

Several clinical studies, albeit largely anecdotal, support the efficacy of IVIG in NMO (Bakker et al., 2004, Can. J. Neurol. Sci. 31, 265-267; Elsone et al., 2014, Mult. Scler. 20, 501-504; Magraner et al., 2013, Neurologia 28, 65-72; Okada et al., 2007, Intern. Med. 46, 1671-1672; Viswanathan et al., 2015, J. Neuroimmunol. 282, 92-96; Wingerchuk 2013, J. Clin. Immunol. 33, Suppl 1: S33-37). A -50% reduction in pathology was previously demonstrated in an experimental mouse model of NMO in which IVIG was administered at a dose that produced serum levels comparable to those in IVIG-treated humans (Ratelade et al., 2014, Mol. Immunol. 62, 103-114). The reduction in NMO pathology by IVIG involved reduced complement- and cell-mediated AQP4-IgG astrocyte injury. Partial efficacy of IVIG was also reported recently in rats administered human NMO patient sera by an intrathecal route (Grünewald et al., 2016, Int. J. Mol. Sci. 17, pii: E1407. doi: 10.3390/ijms17091407).

Interestingly, several of the above mentioned properties could be recapitulated with only the Fc portion of IgG. Various recombinant Fc-based therapeutics are under development, including Fc fusion and multimeric proteins, which have shown efficacy in experimental animal models of arthritis, ITP and inflammatory neuropathy (Anthony et al., 2008, Science 320, 373-376; Czajkowsky et al., 2015, Sci. Rep. 5, 9526; Jain et al., 2012, Arthritis Res. 14, R192; Lin et al., 2007, J. Neuroimmunol. 186, 133-140; Niknami et al., 2013, J. Peripher. Nerv. Syst. 18, 141-152; Thiruppathi et al., 2014, J. Autoimmun. 52, 64-73).

Prospective IVIG replacement proteins comprising multiple Fc domains are described in WO 2008/151088, WO 2012/016073, or WO 2017/019565. While envisaging a variety of different configurations of constructs with multiple Fc fragments, the main class of such constructs disclosed are so-called stradomers, which comprise Fc fragments with multimerization domains such as an IgG2 hinge region. However, no working examples are provided regarding the efficacy of the envisaged multimeric proteins in WO 2008/151088.

Other Fc multimeric constructs with multimerization domains that may be useful in the invention include hexameric constructs where the IgM tailpiece is used to multimerize IgG Fc fragments. For example, WO 2014/060712 discloses an Fc multimeric construct comprising an IgG1 Fc region with a truncated hinge region, a four amino acid linker, and an IgM tailpiece, which multimerizes to predominantly hexameric structure. Mutations at Fc residues 309 and 310 (L309C and H310L) were introduced to mimic the sequence of IgM.

WO 2015/132364 and WO 2015/132365 disclose several Fc multimeric constructs comprising a five amino acid hinge region, an Fc region derived from IgG1, IgG4, or a hybrid of IgG1 and IgG4 CH2 and CH3 domains, and an IgM or IgA tailpiece. The disclosures are directed to improving safety and efficacy of IgG Fc multimers through the introduction of amino acid changes in the Fc regions of the fusion peptides. WO 2016/139365 A1 discloses similar Fc multimeric constructs, however, *in vivo* activity was only tested for some of the constructs in a mouse model for Idiopathic thrombocytopenic purpura (ITP).

Optimized hexameric Fc-µTP constructs were disclosed in WO 2017/129737, which were shown to have several benefits *in vivo, ex vivo,* and *in vitro* over those described previously. Fc-µTP- and Fc-µTP-L309C-bound C1q did not induce cleavage of the complement protein C2, and therefore no C3 convertase was formed (C4b2a). Fc-µTP and Fc-µTP-L309C selectively inhibited activation of the complete classical complement pathway; no interference with the alternative pathway was observed.

The inventors have now surprisingly found that Fc multimers with a multimerization domain, such as Fc-µTP and Fc-µTP-L309C, are effective in the treatment of neuromyelitis optica (NMO). The surprising therapeutic utility of the FC multimeric constructs that has been demonstrated includes:
- Surprisingly inhibit complement-dependent cytotoxicity and antibody-dependent cellular cytotoxicity in an *in vitro* model of NMO.
- Surprisingly inhibit complement-dependent cytotoxicity and regulate pathology ex *vivo* in a spinal cord slice model of NMO.
- Surprisingly prevent cytotoxicity and pathology *in vivo* in a rat model of NMO.

### SUMMARY

The present invention provides an Fc multimeric protein for use in the treatment of neuromyelitis optica, wherein the Fc multimeric protein comprises six IgG Fc fusion monomers as further defined in Claim 1.

In a preferred embodiment of the present invention, the Fc multimer used in the invention comprises six IgG Fc fusion monomers such as those described in WO 2017/129737. Each of the IgG Fc fusion monomers comprises two Fc fusion polypeptide chains and each Fc fusion polypeptide chain comprises an IgG Fc polypeptide and an IgM tailpiece. The Fc multimer is an Fc hexamer, comprising six IgG Fc fusion monomers.

In a preferred embodiment, the Fc fusion polypeptide chain further comprises an IgG hinge region and the Fc fusion polypeptide chain does not comprise a Fab polypeptide.

For example, in one preferred embodiment, the Fc fusion polypeptide chain used in the invention comprises an IgG1 hinge region, an IgG1 Fc domain, and an IgM tailpiece, and does not comprise a Fab polypeptide. In a preferred embodiment, the IgM tailpiece in each Fc fusion polypeptide chain comprises 18 amino acids fused with 232 amino acids at a C-terminus of a constant region of the IgG1 Fc polypeptide. In a further preferred embodiment, the Fc fusion polypeptide chain is SEQ ID NO: 1 and has up to 5 conservative amino acid changes. In a separate preferred embodiment, the Fc fusion polypeptide chain is expressed as SEQ ID NO: 2 (corresponding to SEQ ID NO: 7 of WO 2017/129737), from which the signal peptide is cleaved off during secretion and formation of the mature Fc hexamer.

In a preferred embodiment the Fc fusion polypeptide chain comprises an IgG1 hinge region, an IgG1 Fc domain, and an IgM tailpiece, wherein the IgG1 Fc domain has a cysteine instead of a leucine at position 309 (according to the EU numbering), and wherein the Fc fusion polypeptide does not comprise a Fab polypeptide and the Fc fusion polypeptide chain is SEQ ID NO: 3 (corresponding to SEQ ID NO: 2 of WO 2017/129737). In further preferred embodiment, the Fc fusion polypeptide chain is SEQ ID NO: 3 with up to 5 conservative amino acid changes. In a separate preferred embodiment, the Fc fusion polypeptide chain is expressed as SEQ ID NO: 4 (corresponding to SEQ ID NO: 8 of WO 2017/129737), from which the signal peptide is cleaved off during secretion and formation of the mature Fc hexamer.

In a preferred embodiment, the Fc hexamer blocks complement-dependent cytotoxicity and antibody-dependent cellular cytotoxicity in a concentration-dependent manner in AQP4-expressing Chinese hamster ovary cells *in vitro.*

In a preferred embodiment, the Fc hexamer blocks complement-dependent cytotoxicity initiated in Chinese hamster ovary cells *in vitro* by serum from a seropositive neuromyelitis optica patient.

In a preferred embodiment, the Fc hexamer prevents complement-dependent cytotoxicity and pathology in an ex *vivo* spinal cord slice model of neuromyelitis optica.

In a preferred embodiment, the Fc hexamer prevents complement-dependent cytotoxicity and pathology produced by AQP4-IgG and rat complement in an experimental rat model of neuromyelitis optica. In a preferred embodiment, the Fc hexamer prevents astrocyte injury, demyelination, inflammation and deposition of activated complement in an experimental rat model of neuromyelitis optica.

In a preferred embodiment, the Fc hexamer binds complement component C1q rather than AQP4-IgG or its binding to AQP4. In one embodiment, the Fc hexamer binding to C1q does not induce activation of the complete classical complement pathway.

The Fc hexamer of the invention is provided for use in the treatment of neuromyelitis optica, preferably in a subject by administering a therapeutically effective amount of a pharmaceutical composition of the Fc hexamer to a subject in need thereof.

In a preferred embodiment, the Fc hexamer is administered intravenously or non-intravenously. In one embodiment, the Fc hexamer is administered subcutaneously. In one embodiment, the Fc hexamer is applied orally, or intrathecally, or intrapulmonarily by nebulization.

In a preferred embodiment, the Fc hexamer is administered in an amount ranging from about 10 mg/kg to about 200 mg/kg. In one embodiment, the Fc hexamer is administered in an amount ranging from about 25 mg/kg to about 500 mg/kg. All doses are per kg of bodyweight of the subject to which the Fc hexamer is administered.

In a preferred embodiment, the Fc multimer is produced by expressing polypeptide chains comprising SEQ ID NO: 5, whereby the mature Fc multimer comprises residues 21 to 264 of SEQ ID NO: 5.

### BRIEF DESCRIPTION OF DRAWING(S)

Fig. 1A shows structures of the four Fc preparations tested: (a) clinical-grade IVIG (pooled human IgG), (b) Fc monomers, (c) Fc-µTP hexamers, and (d) Fc- µTP-L309C hexamers.
Fig. 1B shows SDS PAGE of Fc-µTP (left) and Fc-µTP-L309C (right) Fc proteins. Molecular weight markers in kDa are shown.
Fig. 1C shows the size exclusion chromatography (SEC) of Fc-µTP (left) and Fc-µTP-L309C (right). Chromatograms show the normalized U.V. absorbance signals at 280 nm (A280) and the thick bold lines show the molecular weight (in kDa) of material eluted at the time indicated, determined by multi-angle light scattering (MALS).
Fig. 1D shows the asymmetrical flow field-flow fractionation (AF4) of Fc-µTP (left) and Fc-µTP-L309C (right). Chromatograms show the normalized A280 signals and the thick bold lines show the molecular weight (in kDa) of material eluted at the time indicated, determined by MALS.
Fig. 2 shows activation of NFκB by LPS but not the Fc proteins Fc-µTP and Fc-µTP-L309C indicating the lack of endotoxin contamination.
Fig. 3A shows the percent inhibition of complement-dependent cytotoxicity by Fc-µTP and Fc-µTP-L309C hexamers in AQP4-expressing Chinese hamster ovary cells. Prior to addition to cells, the Fc preparations were pre-incubated with 1% or 0.5% human complement.
Fig. 3B shows the percent inhibition of complement-dependent cytotoxicity by Fc monomers and IVIG in AQP4-expressing Chinese hamster ovary cells.
Fig. 3C shows the percent inhibition of complement-dependent cytotoxicity by a 50µg/ml and 100µg/ml concentration of Fc-µTP-L309C hexamer in AQP4-expressing Chinese hamster ovary cells.
Fig. 3D shows the percent inhibition of complement-dependent cytotoxicity by Fc-µTP and Fc-µTP-L309C hexamers in Chinese hamster ovary cells, wherein cytotoxicity was initiated by serum from a seropositive neuromyelitis optica patient.
Fig. 4A shows immunofluorescent staining of ex *vivo* spinal cord slice models of neuromyelitis optica. Spinal cord slices were incubated with (a) control IgG and human complement), (b) AQP4-IgG and human complement, (c) AQP4-IgG, human complement, and Fc-µTP, and (d) AQP4-IgG, human complement, and Fc-µTP-L309C. Astrocyte injury is indicated by loss of AQP4 and GFAP staining, demyelination is indicated by reduced MBP staining, inflammation is indicated by increased Iba-1 staining, and deposition of the complement terminal membrane attack complex is indicated by C5b-9 staining.
Fig. 4B summarizes pathology scores of spinal cord slice models for each treatment group.
Fig. 5 shows the percent inhibition of antibody-dependent cellular cytotoxicity in AQP4-expressing Chinese hamster ovary cells by Fc monomers, IVIG, Fc-µTP and Fc-µTP-L309C hexamers.
Fig. 6A shows immunofluorescent staining of AQP4-expressing Chinese hamster ovary cells. Cells were incubated with AQP4-IgG to determine the potential for binding to AQP4 in the presence of Fc-µTP-L309C hexamer.
Fig. 6B shows immunofluorescent staining of Chinese hamster ovary cells with AQP4-bound AQP4-IgG. Cells were incubated with C1q to determine the potential for binding to AQP4-bound AQP4-IgG in the presence of 20µg/ml Fc-µTP-L309C hexamer, 100µg/ml Fc-µTP-L309C hexamer, and 100µg/ml Fc monomer.

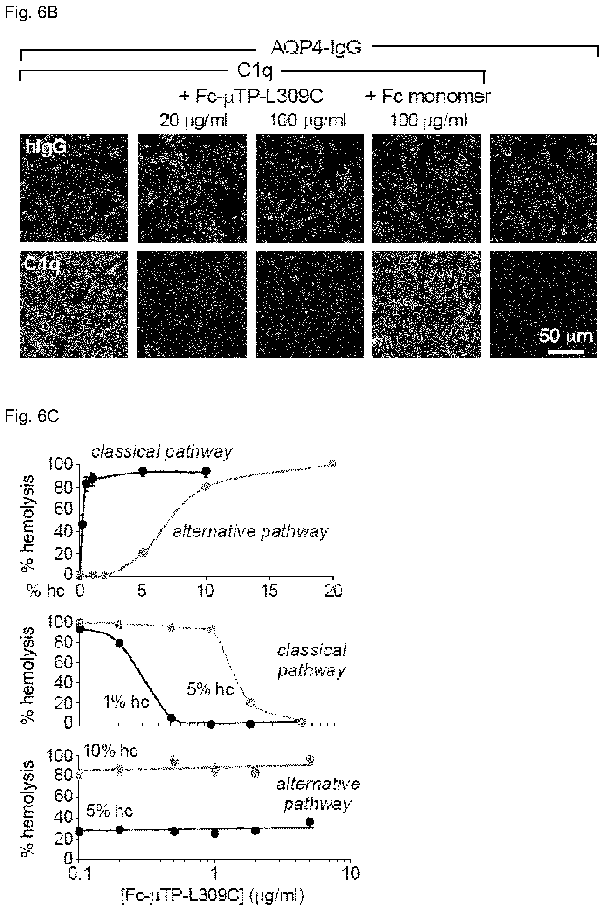

Fig. 6C shows % hemolysis produced by activation of the classical or alternative complement pathways in a model of erythrocyte lysis in the presence of various concentrations of Fc-µTP-L309C hexamer and human complement. (i) shows % hemolysis produced by activation of the classical or alternative complement pathways in the presence of a rising concentration of human complement. (ii) shows % hemolysis produced by activation of the classical complement pathway in the presence of a rising concentration of Fc- µTP-L309C hexamer and either a 1% or 5% concentration of human complement. (iii) shows % hemolysis produced by activation of the alternative complement pathway in the presence of a rising concentration of Fc-µTP-L309C hexamer and either a 5% or 10% concentration of human complement.
Fig. 7A shows the percent inhibition of complement-dependent cytotoxicity in a concentration-dependent manner by Fc-µTP-L309C hexamer in an experimental rat model of neuromyelitis optica in the presence of 1% or 2% rat serum.
Fig. 7B shows the percent inhibition of complement-dependent cytotoxicity *in vitro* in AQP4-expressing Chinese hamster ovary cells. Cells were exposed to serum collected from rats following two-hour administration of 0 mg/kg, 3.125 mg/kg, 6.25 mg/kg, 12.5 mg/kg, 25 mg/kg, and 50 mg/kg doses of Fc-µTP-L309C hexamer.
Fig. 7C shows the time course of percent inhibition of complement-dependent cytotoxicity *in vitro* in AQP4-expressing Chinese hamster ovary cells. Cells were exposed to serum collected from rats at various time points following administration of a 50 mg/kg dose of Fc-µTP-L309C hexamer.

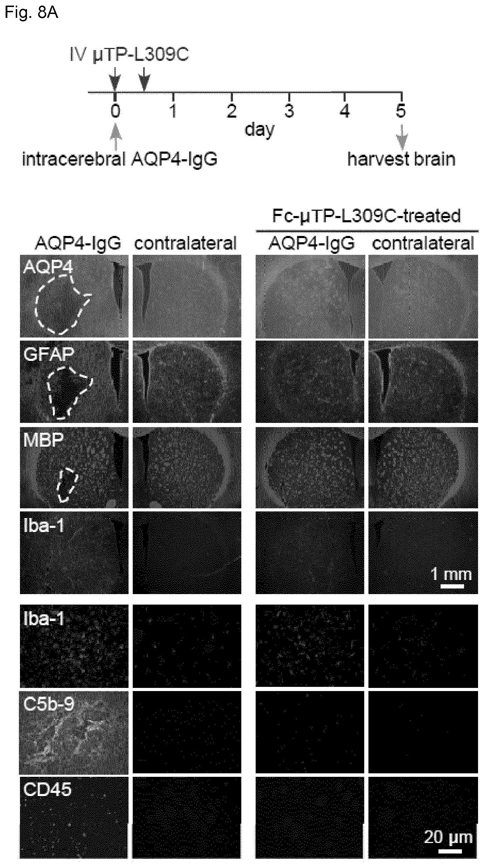

Fig. 8A shows immunofluorescent staining in brains of AQP4-lgG-treated rats. AQP4 was injected intracerebrally in rats. Rats were treated simultaneously with AQP4 and a 50 mg/kg dose of Fc-µTP-L309C hexamer and again with hexamer 12 hours after initial treatment. Brains were harvested and slices were incubated with control IgG or Fc-µTP-L309C. Immunofluorescence of the non-injected contralateral hemisphere is shown for comparison. Astrocyte injury is indicated by loss of AQP4 and GFAP staining, demyelination is indicated by reduced MBP staining, inflammation is indicated by increased Iba-1 and CD45 staining, and deposition of the complement terminal membrane attack complex is indicated by C5b-9 staining.
Fig. 8B shows immunofluorescent staining in brains of rats treated with a large amount of AQP4-IgG. Rats were treated simultaneously with AQP4 and a 50 mg/kg dose of Fc-µTP-L309C hexamer and again with hexamer 12 hours after initial treatment. Brains were harvested and slices were incubated with control IgG or Fc-µTP-L309C. Immunofluorescence of the non-injected contralateral hemisphere is shown for comparison. Astrocyte injury is indicated by loss of AQP4 and GFAP staining and demyelination is indicated by reduced MBP staining.

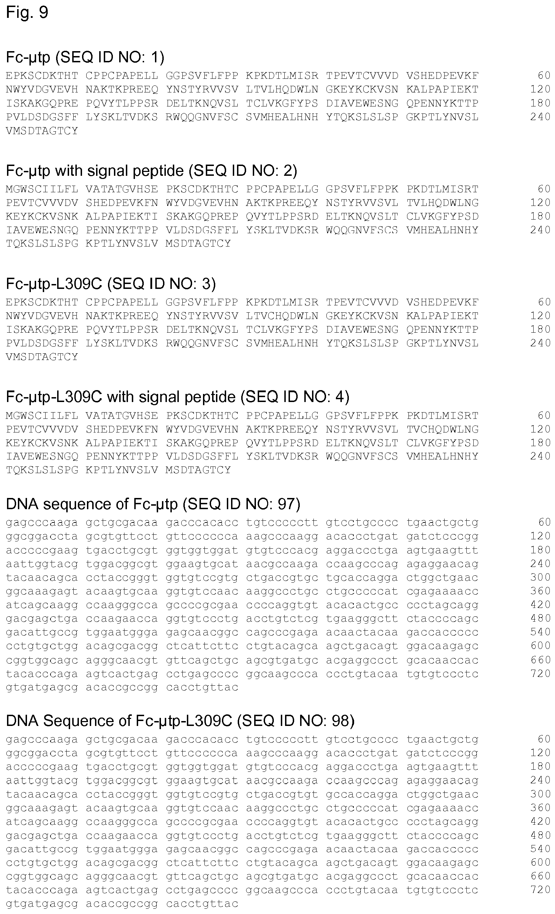

Fig. 9: Sequences from WO2017/129737

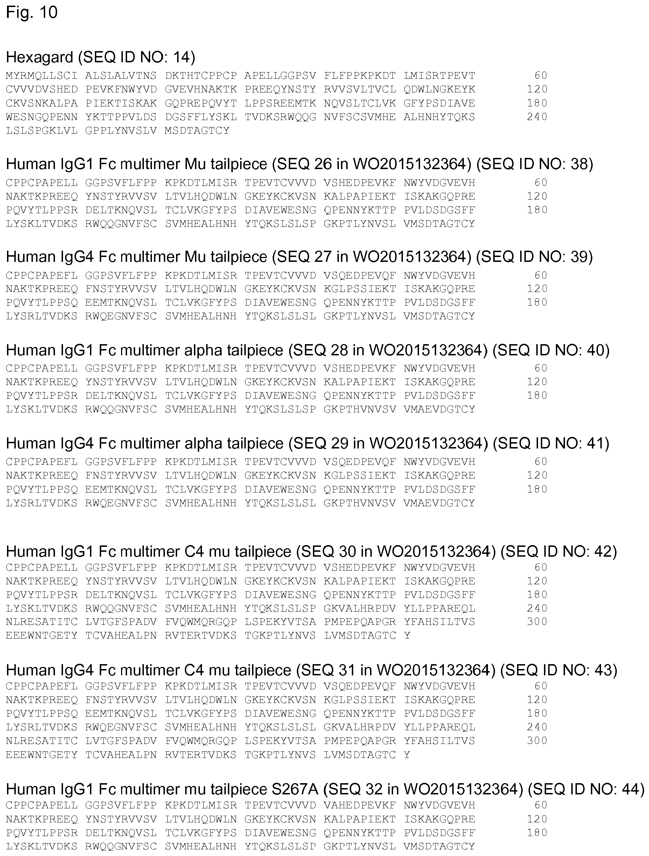

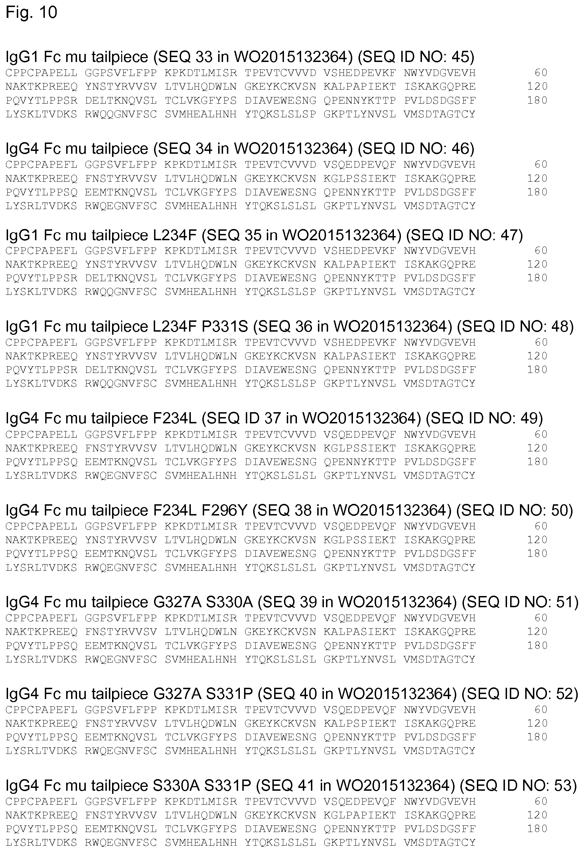

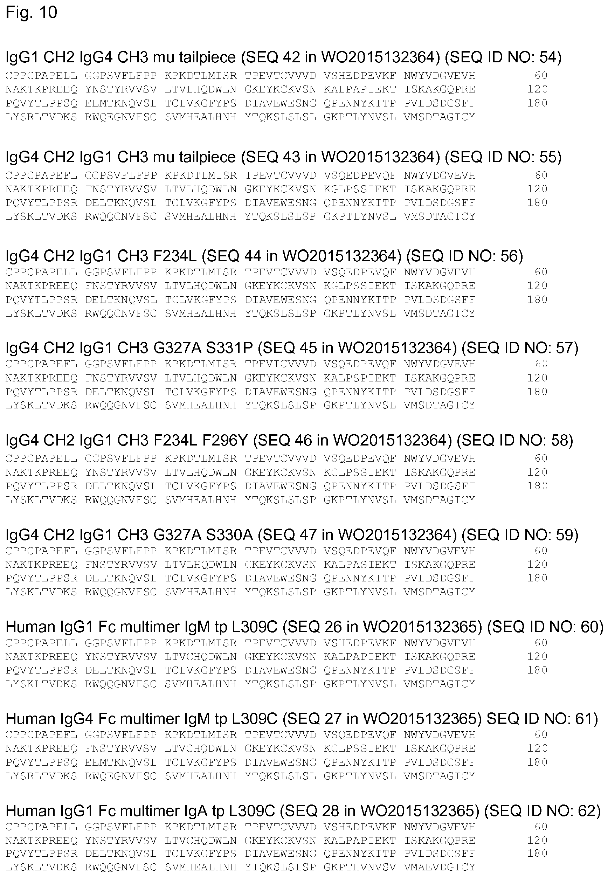

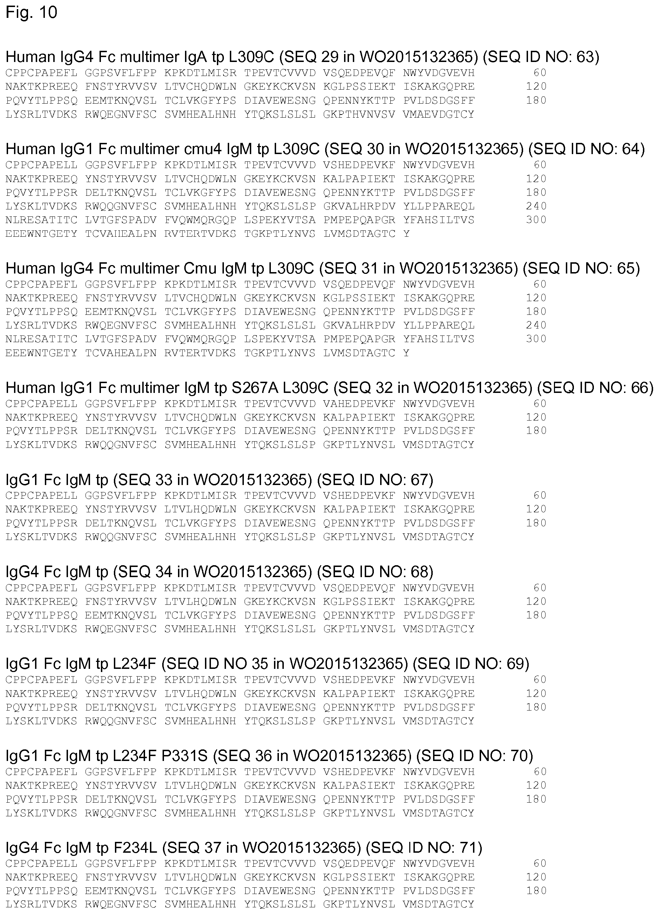

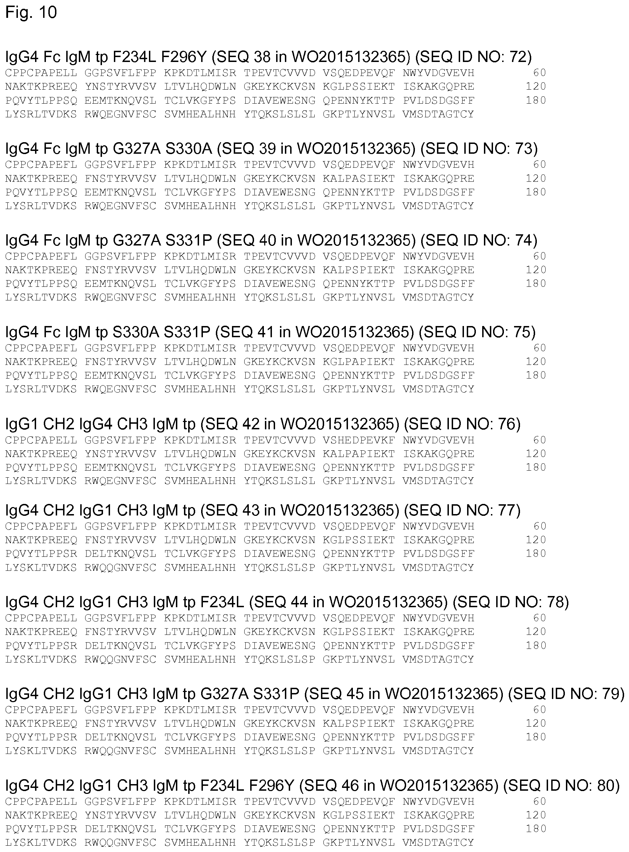

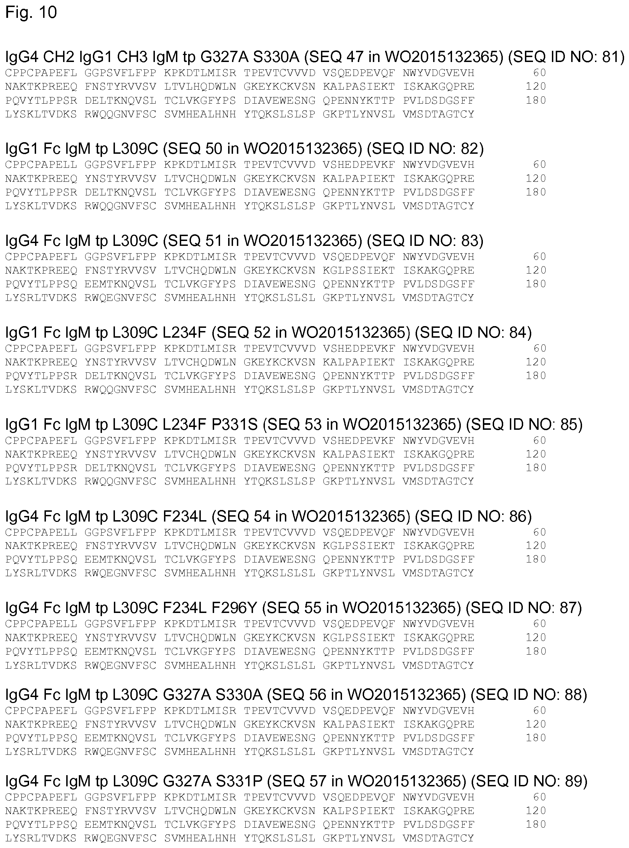

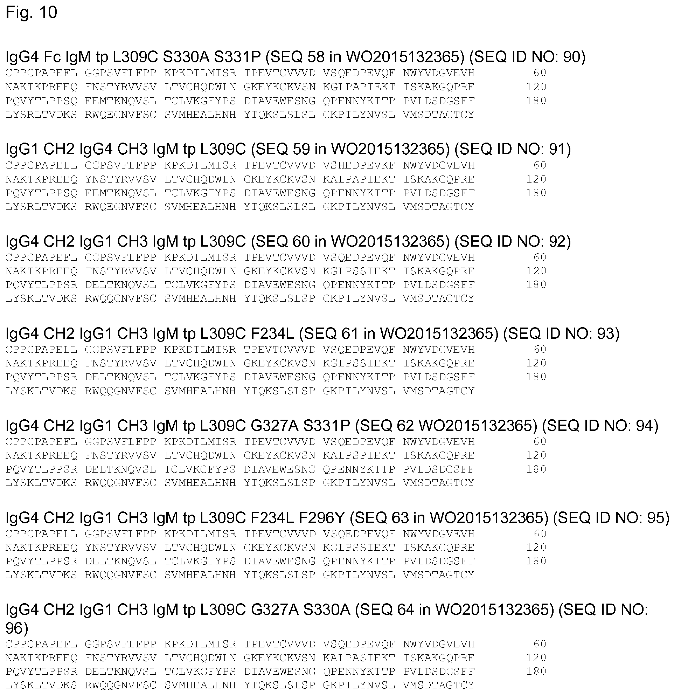

Fig 10: Other hexamer sequences used in embodiments of the invention

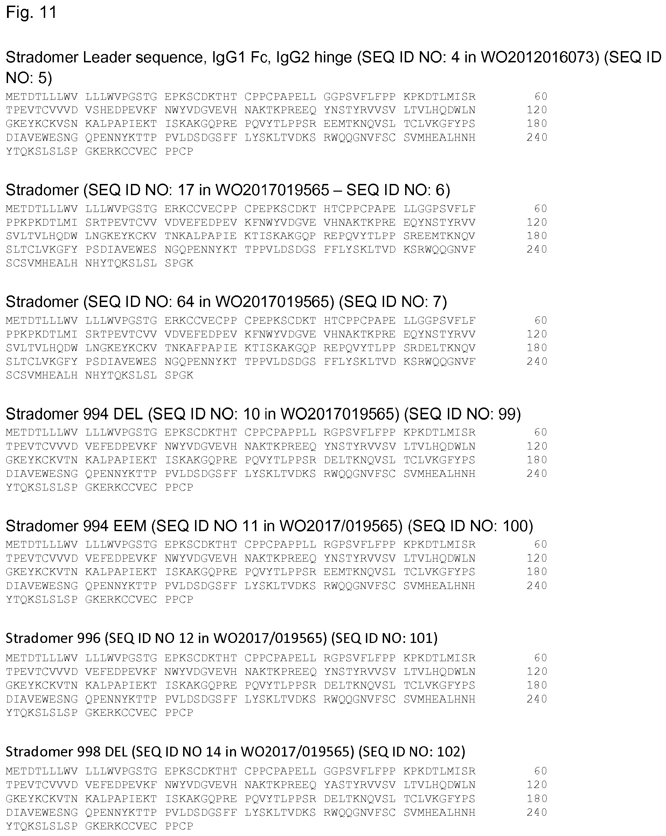

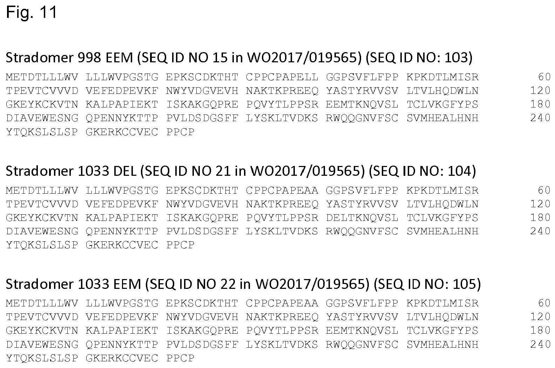

Fig. 11: Stradomer sequences used in embodiments of the invention

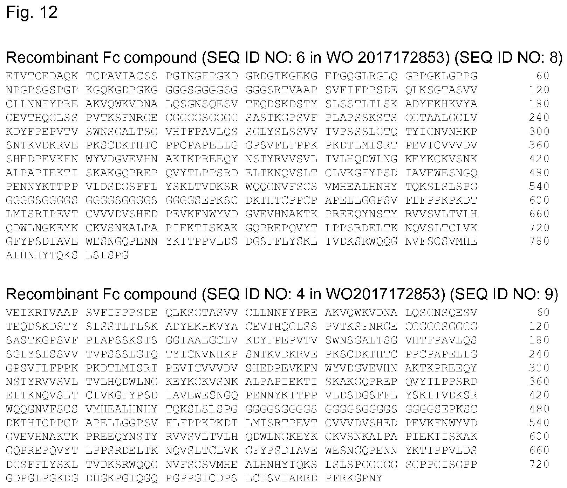

Fig. 12: Recombinant Fc compounds as disclosed in WO 2017/172853, used in embodiments of the present invention

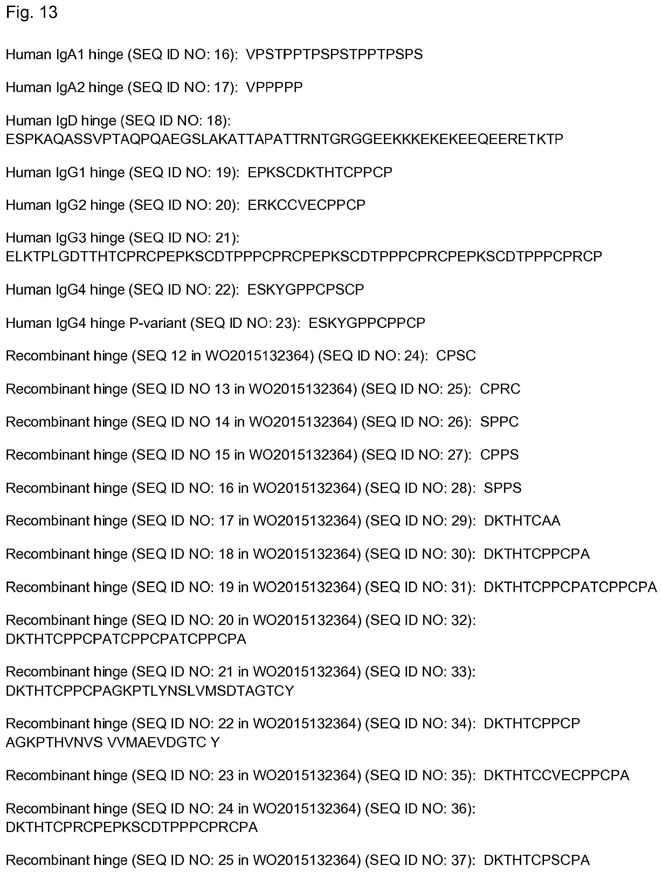

Fig. 13: Examples of suitable hinge regions used in Fc multimers used in embodiments of the invention

### DETAILED DESCRIPTION

The following detailed description and examples illustrate certain embodiments of the present disclosure.

The present invention provides an Fc multimeric protein for use in the treatment of neuromyelitis optica, wherein the Fc multimeric protein comprises six IgG Fc fusion monomers as further defined in Claim 1.

The term "Fc monomer," as used herein, is defined as a portion of an immunoglobulin G (IgG) heavy chain constant region containing the heavy chain CH2 and CH3 domains of IgG, or a variant or fragment thereof. The IgG CH2 and CH3 domains are also referred to as Cy2 and Cy3 domains respectively.

The Fc monomer may be comprised of two identical Fc peptides linked by disulfide bonds between cysteine residues in the N-terminal parts of the peptides. The arrangement of the disulfide linkages described for IgG pertain to natural human antibodies. There may be some variation among antibodies from other vertebrate species, although such antibodies may be suitable in the context of the present invention. The Fc peptides may be produced by recombinant expression techniques and associate by disulfide bonds as occurs in native antibodies. Alternatively, one or more new cysteine residues may be introduced in an appropriate position in the Fc peptide to enable disulfide bonds to form.

In one embodiment, the Fc monomer used in the present invention comprises two identical peptide chains comprising the human IgG1 CH2 and CH3 domains as described in WO 2017/129737.

In another embodiment, the Fc monomer used in the present invention includes the entire CH2 and CH3 domains and is truncated at the N-terminus end of CH2 or the C-terminus end of CH3, respectively as disclosed in WO 2017/129737. Typically, the Fc monomer lacks the Fab polypeptide of the immunoglobulin. The Fab polypeptide is comprised of the CH1 domain and the heavy chain variable region domain.

The Fc monomer used in the present invention may comprise more than the CH2 and CH3 portion of an immunoglobulin. For example, in one embodiment, the monomer includes the hinge region of the immunoglobulin, a fragment or variant thereof, or a modified hinge region. A native hinge region is the region of the immunoglobulin which occurs between CH1 and CH2 domains in a native immunoglobulin. A variant or modified hinge region is any hinge that differs in length and/or composition from the native hinge region. Such hinges can include hinge regions from other species. Other modified hinge regions comprise a complete hinge region derived from an antibody of a different class or subclass from that of the Fc portion. Alternatively, the modified hinge region comprises part of a natural hinge or a repeating unit in which each unit in the repeat is derived from a natural hinge region. In another alternative, the natural hinge region is altered by increasing or decreasing the number of cysteine residues. Other modified hinge regions are entirely non-natural and are designed to possess desired properties such as length, cysteine composition, and flexibility.

A number of modified hinge regions have been described for use in the present invention, for example in US 5,677,425, WO 1998/25971, WO 1999/15549, WO 2005/003169, WO 2005/003170, and WO 2005/003171.

The Fc polypeptide in the Fc multimer used in one embodiment of the present invention possesses a human IgG1 hinge region at its N-terminus. In one embodiment, the hinge region has the sequence of residues 1 to 15 of SEQ ID NO: 1.

The Fc polypeptide chain used in the present invention is expressed comprising a signal peptide as disclosed in WO 2017/129737. The signal peptide directs the secretion of the Fc polypeptide chain and thereafter is cleaved from the remainder of the Fc polypeptide chain.

The Fc polypeptide used in an embodiment of the present invention includes a signal peptide fused to the N-terminus of the hinge region. The signal peptide may have the sequence of residues 1 to 19 of SEQ ID NO: 2; however, the skilled person will be aware that other signal sequences that direct secretion of proteins from mammalian cells may also be used.

In order to improve formation of multimeric structures of the Fc monomers, the Fc peptide is fused to a tailpiece, which causes the monomer units to assemble into a hexamer. The product of the fusion of the Fc peptide to the tailpiece is the "Fc fusion peptide," as used herein. As Fc peptides dimerize to form Fc monomers, Fc fusion peptides likewise dimerize to form Fc fusion monomers.

A "Fc fusion monomer" as used herein therefore comprises two Fc fusion polypeptide chains and each Fc fusion polypeptide chain comprises an IgG Fc polypeptide and an IgM tailpiece.

Suitable tailpieces are derived from IgM. IgM occur naturally in humans as covalent multimers of the common H₂L₂ antibody unit. IgM occurs as a pentamer when it has incorporated a J-chain, or as a hexamer when it lacks a J-chain. The heavy chains of IgM possess a respective 18 amino acid extension to the C-terminal constant domain, known as a tailpiece. This tailpiece includes a cysteine residue that forms a disulfide bond between heavy chains in the polymer, and is believed to have an important role in polymerization. The tailpiece also contains a glycosylation site.

The tailpiece of the present disclosure comprises any suitable amino acid sequence. The tailpiece is a tailpiece found in a naturally occurring antibody, or alternatively, it is a modified tailpiece which differs in length and/or composition from a natural tailpiece. Other modified tailpieces are entirely non-natural and are designed to possess desired properties for multimerization, such as length, flexibility, and cysteine composition.

The tailpiece in the Fc multimer used in an embodiment of the present invention comprises all or part of the 18 amino acid sequence from human IgM as shown in residues 233 to 250 of SEQ ID NO: 1 and in SEQ ID NO: 11. Alternatively, the tailpiece may be a fragment or variant of the human IgM tailpiece.

The tailpiece in the Fc multimer used in one embodiment of the present invention is fused directly to the C-terminus of a constant region of the Fc peptide to form the Fc fusion peptide. Alternatively, the tailpiece is fused to a 232 amino acid segment at the C-terminus of the constant region of the Fc peptide. Alternatively, the tailpiece is fused indirectly by means of an intervening amino acid sequence. For example, a short linker sequence may be provided between the tailpiece and the Fc peptide. A linker sequence may be between 1 and 20 amino acids in length.

Formation of multimeric structures may be further improved by mutating leucine 309 of the Fc portion of the Fc fusion peptide to cysteine. The L309C mutation allows for additional disulfide bond formation between the Fc fusion monomers, which further promotes multimerization of the Fc fusion monomers. The residues of the IgG Fc portion are numbered according to the EU numbering system for IgG, described in Edelman GM et al (1969), Proc Natl Acad Sci 63, 78-85; see also Kabat et al., 1983, Sequences of proteins of immunological interest, US Department of Health and Human Services, National Institutes of Health, Washington, DC. Leu 309 of IgG corresponds by sequence homology to Cys 414 in Cµ3 domain of IgM and Cys 309 in the Cα2 domain of IgA.

Other mutations additionally, or alternatively, are introduced in the Fc fusion peptide to achieve desirable effects. The term "mutation," as used herein, includes a substitution, addition, or deletion of one or more amino acids. In some embodiments, as described in WO 2017/129737, the Fc fusion peptide comprises up to 20, up to 10, up to 5, or up to 2 amino acid mutations.

The mutations in the Fc multimer used in one embodiment of the present invention are conservative amino acid changes as described in WO 2017/129737. The term "conservative amino acid changes," as used herein, refers to the change of an amino acid to a different amino acid with similar biochemical properties, such as charge, hydrophobicity, structure, and/or size. The Fc fusion peptide used in an embodiment of the present invention comprises up to 20, up to 10, up to 5, or up to 2 conservative amino acid changes. For example, the Fc fusion peptide comprises up to 5 conservative amino acid changes.

A conservative amino acid change includes a change amongst the following groups of residues: Val, Ile, Leu, Ala, Met; Asp, Glu; Asn, Gln; Ser, Thr, Gly, Ala; Lys, Arg, His; and Phe, Tyr, Trp.

A "variant," when used herein to describe a peptide, protein, or fragment thereof, may have modified amino acids. Suitable modifications include acetylation, glycosylation, hydroxylation, methylation, nucleotidylation, phosphorylation, ADP-ribosylation, and other modifications known in the art. Such modifications may occur post-translationally where the peptide is made by recombinant techniques. Otherwise, modifications may be made to synthetic peptides using techniques known in the art. Modifications may be included prior to incorporation of an amino acid into a peptide. Carboxylic acid groups may be esterified or may be converted to an amide, an amino group may be alkylated, for example methylated. A variant may also be modified post-translationally, for example to remove or add carbohydrate side-chains or individual sugar moieties.

The term "Fc multimer," as used herein, describes six polymerized Fc fusion monomers. An Fc multimer comprises six Fc fusion monomers, producing Fc hexamers. Fc fusion monomers naturally associate into polymers having different numbers of monomer units.

As disclosed in WO 2017/129737, the majority of Fc multimer is an Fc hexamer. As used herein, the term "majority" refers to greater than 50%, greater than 60%, greater than 70%, greater than 80%, or greater than 90%. In one embodiment, greater than 80% of the Fc multimer is an Fc hexamer.

If Fc multimers containing a specific number of monomers are required, Fc multimers can be separated according to molecular size, for example by gel filtration (size exclusion chromatography).

### Polynucleotides (not part of the invention)

The disclosure further relates to a polynucleotide encoding an Fc fusion peptide for an Fc multimer. The term "polynucleotide(s)" generally refers to any polyribonucleotide or polydeoxyribonucleotide that may be unmodified RNA or DNA or modified RNA or DNA. The polynucleotide can be single- or double-stranded DNA, single or double-stranded RNA. As used herein, the term "polynucleotide(s)" also includes DNAs or RNAs that comprise one or more modified bases and/or unusual bases, such as inosine. It will be appreciated that a variety of modifications may be made to DNA and RNA that serve many useful purposes known to those of skill in the art. The term "polynucleotide(s)" as it is employed herein embraces such chemically, enzymatically, or metabolically modified forms of polynucleotides, as well as the chemical forms of DNA and RNA characteristic of viruses and cells, including, for example, simple and complex cells.

The skilled person would understand that, due to the degeneracy of the genetic code, a given polypeptide can be encoded by different polynucleotides. These "variants" are encompassed by the Fc multimers disclosed herein.

The polynucleotides of the Fc multimers may be an isolated polynucleotide. The term "isolated" polynucleotide refers to a polynucleotide that is substantially free from other nucleic acid sequences, such as and not limited to other chromosomal and extrachromosomal DNA and RNA. In one embodiment, the isolated polynucleotides are purified from a host cell. Conventional nucleic acid purification methods known to skilled artisans may be used to obtain isolated polynucleotides. The term also includes recombinant polynucleotides and chemically synthesized polynucleotides.

Another aspect of the disclosure (not a part of the invention) is a plasmid or vector comprising a polynucleotide according to the disclosure. As disclosed in WO 2017/129737, the plasmid or vector comprises an expression vector. In one embodiment, the vector is a transfer vector for use in human gene therapy. Another aspect of the disclosure (not a part of the invention) is a host cell comprising a polynucleotide, a plasmid, or vector of the disclosure.

The host cell of the disclosure is employed in a method of producing an Fc multimer. The method comprises:
(a) culturing host cells of the disclosure under conditions such that the desired insertion protein is expressed; and
(b) optionally recovering the desired insertion protein from the host cells or from the culture medium.

The Fc multimers can be purified to ≥80% purity, ≥90% purity, ≥95% purity, ≥99% purity, or ≥99.9% purity with respect to contaminating macromolecules, for example other proteins and nucleic acids, and free of infectious and pyrogenic agents. An isolated Fc multimer of the disclosure may be substantially free of other, non-related polypeptides.

In certain embodiments, examples of the Fc multimers of the invention include polymeric proteins comprising six polypeptide monomer units as described in WO 2014/060712, wherein each polypeptide monomer unit comprises an Fc receptor binding portion comprising two immunoglobulin G heavy chain constant regions, wherein each immunoglobulin G heavy chain constant region comprises a cysteine residue which is linked via a disulfide bond to a cysteine residue of an immunoglobulin G heavy chain constant region of an adjacent polypeptide monomer unit, wherein the polymeric protein does not comprise a further immunomodulatory portion or an antigen portion that causes antigen-specific immunosuppression when administered to a mammalian subject. In certain aspects, the two immunoglobulin G heavy chain constant regions are linked via a polypeptide linker as a single chain Fc. The polypeptide monomer unit consists of an Fc receptor binding portion and a tailpiece region fused to the two immunoglobulin G heavy chain constant regions, which facilitates assembly of the monomer units into a polymer.

In another embodiment, each of the immunoglobulin G heavy chain constant regions comprises an amino acid sequence of a mammalian heavy chain constant region, preferably a human heavy chain constant region; or variant thereof. A suitable human IgG subtype is IgG1.

The Fc receptor binding portion may comprise more than the Fc portion of an immunoglobulin. For example, as described in WO 2014/060712, it may include the hinge region of the immunoglobulin which occurs between CH1 and CH2 domains in a native immunoglobulin. For certain immunoglobulins, the hinge region is necessary for binding to Fc receptors. Preferably, the Fc receptor binding portion lacks a CH1 domain and heavy chain variable region domain (VH). The Fc receptor binding portion may be truncated at the C- and/or N- terminus compared to the Fc portion of the corresponding immunoglobulin. The polymeric protein is formed by virtue of each immunoglobulin G heavy chain constant region comprising a cysteine residue which is linked via a disulfide bond to a cysteine residue of an immunoglobulin G heavy chain constant region of an adjacent polypeptide monomer unit. The ability of monomer units based on IgG heavy chain constant regions to form polymers may be improved by modifying the parts of the IgG heavy chain constant regions to be more like the corresponding parts of IgM or IgA. Each of the immunoglobulin heavy chain constant regions or variants thereof is an IgG heavy chain constant region comprising an amino acid sequence which comprises a cysteine residue at position 309 and, preferably, a leucine residue at position 310.

Each polypeptide monomer unit comprises a tailpiece region fused to each of the two immunoglobulin G heavy chain constant regions, wherein the tailpiece region of each polypeptide monomer unit facilitates the assembly of the monomer units into a polymer such as described in WO 2014/060712. For example, the tailpiece region is fused C-terminal to each of the two immunoglobulin heavy chain constant regions. The tailpiece region is an IgM tailpiece, or fragment or variant thereof.

In one embodiment of the Fc multimer of the invention, an intervening amino acid sequence may be provided between the heavy chain constant region and the tailpiece, or the tailpiece may be fused directly to the C-terminus of the heavy chain constant region such as disclosed in WO 2014/060712. For example, a short linker sequence may be provided between the tailpiece region and immunoglobulin heavy chain constant region. Typical linker sequences are of between 1 and 20 amino acids in length, typically 2, 3, 4, 5, 6 or up to 8, 10, 12, or 16 amino acids in length. A suitable linker to include between the heavy chain region and tailpiece region encodes for Leu-Val-Leu-Gly (SEQ ID NO: 10). A preferred tailpiece region is the tailpiece region of human IgM, which is PTLYNVSLVMSDTAGTCY (SEQ ID NO: 11) (Rabbitts TH et al, 1981. Nucleic Acids Res. 9 (18), 4509- 4524; Smith et al (1995) J Immunol 154: 2226-2236). This tailpiece may be modified at the N-terminus by substituting Pro for the initial Thr. This does not affect the ability of the tailpiece to promote polymerization of the monomer. Further suitable variants of the human IgM tailpiece are described in Sorensen et al (1996) J Immunol 156: 2858-2865. A further IgM tailpiece sequence is GKPTLYNVSLIMSDTGGTCY (SEQ ID NO: 12) from rodents. A tailpiece region, which is not part of the present invention, is the tailpiece region of human IgA, which is PTHVNVSVVMAEVDGTCY (SEQ ID NO: 13). Other suitable tailpieces from IgM of other species, or even synthetic sequences which facilitate assembly of the monomer units into a polymer, may be used. It is not necessary to use an immunoglobulin tailpiece from the same species from which the immunoglobulin heavy chain constant regions are derived, although it is preferred to do so.

In certain aspects, the Fc multimer of the invention does not activate the classical pathway of complement, although it may be capable of binding to C1q. The polymeric protein typically has a diameter of about 20 nm, such as from 15 to 25 nm or up to 30 nm. As a consequence of the molecular size and diameter, the polymeric protein typically has a good degree of tissue penetration.

The preferred Fc multimer described in WO 2014/060712 is the hexamer of SEQ ID NO: 14 (SEQ ID NO: 8 in WO 2014/060712), from which the signal peptide is cleaved off during secretion so that the mature product comprises residues 21 to 269 of SEQ ID NO: 14.

In certain embodiments of the present invention, the Fc multimers used are those described in WO 2015/132364, which relates to multimeric fusion proteins which bind to human Fc receptors. Fusion proteins comprise a tailpiece, in the absence of a cysteine residue at position 309.

The multimeric fusion proteins comprise six polypeptide monomer units, wherein each polypeptide monomer unit comprises an antibody Fc-domain comprising two heavy chain Fc-regions. Each heavy chain Fc-region comprises any amino acid residue other than cysteine at position 309, and is fused at its C-terminal to a tailpiece which causes the monomer units to assemble into a multimer. Each polypeptide monomer unit does not comprise an antibody variable region.

In certain aspects, the multimeric fusion proteins of the invention further comprise a fusion partner, which can be an antigen, pathogen-associated molecular pattern (PAMP), drug, ligand, receptor, cytokine or chemokine. The fusion partner is fused to the N-terminus of each heavy chain Fc-region either directly or indirectly by means of an intervening amino acid sequence, such as a hinge. A short linker sequence, alternatively, may be provided between the fusion partner and the heavy chain Fc-region.

In other aspects, the multimeric fusion proteins do not comprise one or more antibody variable regions. Typically, the molecules do not comprise either a VH or a VL antibody variable region. In certain further aspects, the multimeric fusion proteins of WO 2015/132364 do not comprise a Fab fragment.

Each polypeptide monomer unit of the multimeric fusion protein comprises an antibody Fc-domain, which may be derived from any suitable species, including humans, for instance. The antibody Fc-domain is derived from IgG (including subclasses IgG1, IgG2, IgG3 and IgG4).

The antibody Fc-domain comprises two polypeptide chains, each referred to as a heavy chain Fc-region. The two heavy chain Fc regions dimerize to create the antibody Fc-domain. The two heavy chain Fc regions within the antibody Fc domain may be different from one another but will typically be the same.

Typically, each heavy chain Fc-region comprises or consists of two or three heavy chain constant domains. IgG is composed of two heavy chain constant domains (CH2 and CH3).

Thus, the heavy chain Fc region in the Fc multimer used in one embodiment of the present invention comprises a CH3 domain derived from IgG1 such as disclosed in WO 2015/132364. In a separate embodiment, the heavy chain Fc region comprises a CH2 domain derived from IgG4 and a CH3 domain derived from IgG1. In certain embodiments, the heavy chain Fc region comprises an arginine residue at position 355. In other embodiments, the heavy chain Fc region comprises a cysteine residue at position 355.

The IgM tailpiece of the multimeric fusion proteins may comprise any suitable amino acid sequence. It may be a tailpiece found in a naturally occurring antibody, or alternatively, it may be a modified tailpiece which differs in length and/or composition from a natural tailpiece. Other modified tailpieces may be entirely synthetic and may be designed to possess desired properties for multimerization, such as length, flexibility and cysteine composition. The tailpiece may be derived from any suitable species, including humans.

The tailpiece may comprise all or part of an 18 amino acid tailpiece sequence from human IgM as shown in SEQ ID NO: 11.

The tailpiece may be fused directly to the C-terminus of the heavy chain Fc-region, or, alternatively, indirectly by means of an intervening amino acid sequence. A short linker sequence, for instance, may be provided between the tailpiece and the heavy chain Fc-region.

The tailpiece may include variants or fragments of the native sequences described above. A variant of an IgM tailpiece typically has an amino acid sequence which is identical to the native sequence in 8, 9, 10, 11, 12, 13, 14, 15, 16, or 17 of the 18 amino acid positions. A fragment typically comprises 8, 9, 10, 11, 12, 13, 14, 15, 16, or 17 amino acids.

Each heavy chain Fc-region in the Fc multimer used in an embodiment of the present invention may, optionally, possess a native or a modified hinge region at its N-terminus. The types of modified hinge regions that can be incorporated in the Fc multimers used in the present invention are disclosed in WO 2015/132364. For example, the heavy chain Fc-region possesses an intact hinge region at its N-terminus. In certain aspects, as disclosed in WO 2015/132364, the heavy chain Fc-region and hinge region are derived from !gG4 and the hinge region comprises the mutated sequence CPPC (SEQ ID NO: 15).

Examples of suitable hinge sequences are shown in SEQ ID Nos: 15 to 37.

Accordingly, in a specific embodiment, a multimeric fusion protein used in the present invention consists of six polypeptide monomer units, wherein each polypeptide monomer unit consists of an antibody Fc-domain and a IgM tailpiece region, wherein each antibody Fc domain consists of two heavy chain Fc-regions in which the amino acid residue at position 309 is any amino acid residue other than cysteine, and, optionally, each heavy chain Fc region possesses a hinge region at the N- terminus, and wherein the tailpiece region is fused to the C-terminus of each heavy chain Fc region and causes the monomer units to assemble into a multimer.

Similarly, the polypeptide monomer units within a particular multimeric fusion protein may be the same as one another or different from one another.

In certain embodiments, a polypeptide chain of a polypeptide monomer unit comprises an amino acid sequence as provided in SEQ ID NOs: 38 to 59 with a tailpiece sequence, optionally with an alternative hinge.

In another example, a multimeric fusion protein used in the present invention comprises or consists of six polypeptide monomer units, wherein each polypeptide monomer unit comprises two identical polypeptide chains, each polypeptide chain comprising of the sequence given in any one of the above SEQ ID NOs: 38 to 59 (SEQ ID NOs 26 to 47 of WO 2015/132364), and wherein each polypeptide monomer unit does not comprise an antibody variable region.

In certain embodiments, the hexameric fusion proteins of the invention comprise one or more mutations which decrease cytokine release and/or decrease platelet activation and/or decrease C1q binding and/or increase the potency of inhibition of macrophage phagocytosis of antibody-coated target cells and/or alter binding to one or more Fc-receptors when compared to unmodified multimeric fusion proteins.

In certain embodiments, the hexameric Fc multimers as defined in Claim 1 of present invention are those described in WO 2015/132365, which relates to multimeric fusion proteins which bind to human Fc receptors.

In certain embodiments, the multimeric fusion proteins of the invention further comprise a fusion partner, as described above. In other embodiments, the multimeric fusion proteins of the invention do not comprise one or more antibody variable regions or a Fab fragment, as described above. In one embodiment, each polypeptide monomer unit of the multimeric fusion protein comprises an antibody Fc-domain with heavy chain Fc regions, as described above. The tailpieces, modified hinge regions, and polypeptide monomer units of the multimeric fusion proteins of the present invention comprise the features described above.

The multimeric fusion protein used in a specific embodiment of the present invention consists of six IgG Fc fusion monomer units, wherein each polypeptide monomer unit, i.e. each Fc fusion monomer, consists of an antibody Fc-domain, i.e. consists of two Fc fusion polypeptide chains, and the tailpiece region, as further defined in Claim 1, wherein each antibody Fc domain consists of two heavy chain Fc-regions in which the amino acid residue at position 309 in each heavy chain Fc region is a cysteine residue and each heavy chain Fc region comprises at least one further mutation which alters FcR binding and/or complement binding and, optionally, each heavy chain Fc region possesses a hinge region at the N-terminus, and wherein the tailpiece region is fused to the C-terminus of each heavy chain Fc region and causes the monomer units to assemble into a multimer.

In certain embodiments, the polypeptide chains of polypeptide monomer units comprise amino acid sequences as described above.

In another example, each polypeptide monomer unit comprises two identical polypeptide chains each polypeptide chain comprising or consisting of the sequence given in any one of the SEQ ID NOs 60 to 96 (corresponding to SEQ ID NOs: 26 to 32 and 50 to 64 of WO 2015/132365), and wherein each polypeptide monomer unit does not comprise an antibody variable region.

In certain embodiments, as taught in WO 2015/132365, the multimeric fusion proteins used in the invention comprise one or more mutations which enable such functions as described above.

The Fc multimeric proteins of the invention are provided for use in the treatment of neuromyelitis optica in a subject in need thereof. The treatment comprises administering to said subject a therapeutically effective amount of the Fc multimer. In another embodiment, the treatment comprises administering to said subject a therapeutically effective amount of a polynucleotide of the disclosure or a plasmid or vector of the disclosure.

### Expression of the proposed Fc multimers (not part of the invention)

The production of recombinant proteins at high levels in suitable host cells requires the assembly of the above-mentioned modified cDNAs into efficient transcriptional units together with suitable regulatory elements in a recombinant expression vector that can be propagated in various expression systems according to methods known to those skilled in the art. Efficient transcriptional regulatory elements could be derived from viruses having animal cells as their natural hosts or from the chromosomal DNA of animal cells. For example, promoter-enhancer combinations derived from the Simian Virus 40, adenovirus, BK polyoma virus, human cytomegalovirus, or the long terminal repeat of Rous sarcoma virus, or promoter-enhancer combinations including strongly constitutively transcribed genes in animal cells like beta-actin or GRP78 can be used. In order to achieve stable high levels of mRNA transcribed from the cDNAs, the transcriptional unit should contain in its 3'-proximal part a DNA region encoding a transcriptional termination-polyadenylation sequence. For example, this sequence can be derived from the Simian Virus 40 early transcriptional region, the rabbit beta globin gene, or the human tissue plasminogen activator gene.

The cDNAs can then be integrated into the genome of a suitable host cell line for expression of the Fc multimer. In some embodiments, this cell line should be an animal cell-line of vertebrate origin in order to ensure correct folding, disulfide bond formation, asparagine-linked glycosylation and other post-translational modifications as well as secretion into the cultivation medium. Examples of other post-translational modifications are tyrosine O-sulfation and proteolytic processing of the nascent polypeptide chain. Examples of cell lines that can be used are monkey COS-cells, mouse L-cells, mouse C127-cells, hamster BHK-21 cells, human embryonic kidney 293 cells, and hamster CHO-cells.

The recombinant expression vector encoding the corresponding cDNAs can be introduced into an animal cell line in several different ways. For example, recombinant expression vectors can be created from vectors based on different animal viruses. Examples of these are vectors based on baculovirus, vaccinia virus, adenovirus, and bovine papilloma virus.

The transcription units encoding the corresponding DNAs can also be introduced into animal cells together with another recombinant gene which may function as a dominant selectable marker in these cells in order to facilitate the isolation of specific cell clones which have integrated the recombinant DNA into their genome. Examples of this type of dominant selectable marker genes are TN4 amino glycoside phosphotransferase, conferring resistance to geneticin (G418), hygromycin phosphotransferase, conferring resistance to hygromycin, and puromycin acetyl transferase, conferring resistance to puromycin. The recombinant expression vector encoding such a selectable marker can reside either on the same vector as the one encoding the cDNA of the desired protein, or it can be encoded on a separate vector which is simultaneously introduced and integrated to the genome of the host cell, frequently resulting in a tight physical linkage between the different transcription units.

Other types of selectable marker genes which can be used together with the cDNA of the desired protein are based on various transcription units encoding dihydrofolate reductase (dhfr). After introduction of this type of gene into cells lacking endogenous dhfr-activity, for example CHO-cells (DUKX-B11, DG-44), it will enable these to grow in media lacking nucleosides. An example of such a medium is Ham's F12 without hypoxanthine, thymidine, and glycine. These dhfr-genes can be introduced together with the cDNA encoding the IgG Fc fusion monomer into CHO-cells of the above type, either linked on the same vector on different vectors, thus creating dhfr-positive cell lines producing recombinant protein.

If the above cell lines are grown in the presence of the cytotoxic dhfr-inhibitor methotrexate, the new cell lines resistant to methotrexate will emerge. These cell lines may produce recombinant protein at an increased rate due to the amplified number of linked dhfr and the desired protein's transcriptional units. When propagating these cell lines in increasing concentrations of methotrexate (1-10,000 nM), new cell lines can be obtained which produce the desired protein at very high rate.

The above cell lines producing the desired protein can be grown on a large scale, either in suspension culture or on various solid supports. Examples of these supports are micro carriers based on dextran or collagen matrices, or solid supports in the form of hollow fibers or various ceramic materials. When grown in cell suspension culture or on micro carriers the culture of the above cell lines can be performed either as a bath culture or as a perfusion culture with continuous production of conditioned medium over extended periods of time. Thus, according to the present disclosure, the above cell lines are well suited for the development of an industrial process for the production of the desired recombinant proteins.

### Purification and Formulation (not part of the invention)

The recombinant protein can be concentrated and purified by a variety of biochemical and chromatographic methods, including methods utilizing differences in size, charge, hydrophobicity, solubility, specific affinity, etc., between the desired protein and other substances in the host cell or cell cultivation medium.

An example of such purification is the adsorption of the recombinant protein to a monoclonal antibody directed to e.g. the Fc portion of the Fc multimer or another Fc-binding ligand (e.g. protein A or protein G), which is immobilized on a solid support. After adsorption of the Fc multimer to the support, washing and desorption, the protein can be further purified by a variety of chromatographic techniques based on the above properties. The order of the purification steps is chosen, for example, according to capacity and selectivity of the steps, stability of the support or other aspects. Purification steps, for example, may be, but are not limited to, ion exchange chromatography steps, immune affinity chromatography steps, affinity chromatography steps, dye chromatography steps, and size exclusion chromatography steps.

In order to minimize the theoretical risk of virus contaminations, additional steps may be included in the process that allow effective inactivation or elimination of viruses. For example, such steps may include heat treatment in the liquid or solid state, treatment with solvents and/or detergents, radiation in the visible or UV spectrum, gamma-radiation, partitioning during the purification, or virus filtration (nano filtration).

The Fc multimers described herein can be formulated into pharmaceutical preparations for therapeutic use. The components of the pharmaceutical preparation may be resuspended or dissolved in conventional physiologically compatible aqueous buffer solutions to which there may be added, optionally, pharmaceutical excipients to provide the pharmaceutical preparation. The components of the pharmaceutical preparation may already contain all necessary pharmaceutical, physiologically compatible excipients and may be dissolved in water for injection to provide the pharmaceutical preparation.

Such pharmaceutical carriers and excipients as well as the preparation of suitable pharmaceutical formulations are well known in the art (see for example, "Pharmaceutical Formulation Development of Peptides and Proteins," Frokjaer et al., Taylor & Francis (2000) or "Handbook of Pharmaceutical Excipients," 3rd edition, Kibbe et al., Pharmaceutical Press (2000)). In certain embodiments, a pharmaceutical composition can comprise at least one additive such as a bulking agent, buffer, or stabilizer. Standard pharmaceutical formulation techniques are well known to persons skilled in the art (see, e.g., 2005 Physicians' Desk Reference®, Thomson Healthcare: Monvale, NJ, 2004; Remington: The Science and Practice of Pharmacy, 20th ed., Gennaro et al., Eds. Lippincott Williams & Wilkins: Philadelphia, PA, 2000). Suitable pharmaceutical additives include, e.g., sugars like mannitol, sorbitol, lactose, sucrose, trehalose, or others, amino acids like histidine, arginine, lysine, glycine, alanine, leucine, serine, threonine, glutamic acid, aspartic acid, glutamine, asparagine, phenylalanine, proline, or others, additives to achieve isotonic conditions like sodium chloride or other salts, stabilizers like Polysorbate 80, Polysorbate 20, Polyethylene glycol, propylene glycol, calcium chloride, or others, physiological pH buffering agents like Tris(hydroxymethylaminomethan), and the like. In certain embodiments, the pharmaceutical compositions may contain pH buffering reagents and wetting or emulsifying agents. In further embodiments, the compositions may contain preservatives or stabilizers. In particular, the pharmaceutical preparation comprising the Fc multimers described herein may be formulated in lyophilized or stable soluble form. The Fc multimers factor may be lyophilized by a variety of procedures known in the art. Lyophilized formulations are reconstituted prior to use by the addition of one or more pharmaceutically acceptable diluents such as sterile water for injection or sterile physiological saline solution or a suitable buffer solution.

The composition(s) of the pharmaceutical preparation of Fc multimer may be delivered to the individual by any pharmaceutically suitable means. Various delivery systems are known and can be used to administer the composition by any convenient route. The composition(s) of the pharmaceutical preparation of the Fc multimer can be formulated for intravenous or non-intravenous injection or for enteral (e.g., oral, vaginal, or rectal) delivery according to conventional methods. For non-intravenous administration, the composition(s) of the Fc multimer can be formulated for subcutaneous, intramuscular, intra-articular, intraperitoneal, intracerebral, intrathecal, intrapulmonary (e.g. nebulized), intranasal, intradermal, peroral or transdermal administration. In one embodiment, the composition(s) of the Fc multimer are formulated for intravenous injection. In other embodiments, the composition(s) of the Fc multimer are formulated for subcutaneous, intramuscular, or transdermal administration, preferably for subcutaneous administration. The formulations can be administered continuously by infusion or by bolus injection. Some formulations can encompass slow release systems.

The composition(s) of the pharmaceutical preparation of Fc multimer is/are administered to patients in a therapeutically effective dose. The term "therapeutically effective," as used herein, describes a dose that is sufficient to produce the desired effects, preventing or lessening the severity or spread of neuromyelitis optica, or to exhibit a detectable therapeutic or preventative effect, without teaching a dose which produces intolerable adverse side effects. The exact dose depends on many factors as, for example, the formulation and mode of administration. The therapeutically effective amount can be initially estimated in cell culture assays or in animal models, for example rodent, rabbit, dog, pig, or primate models. Such information can then be used to determine useful doses and routes for administration in humans.

In one embodiment, the dose of the Fc multimer for one intravenous or one non-intravenous injection is less than 1,000 mg/kg body weight, less than 800 mg/kg body weight, less than 600 mg/kg body weight, less than 400 mg/kg body weight, less than 200 mg/kg body weight, or less than 100 mg/kg body weight. For example, in one embodiment, the dose of Fc multimer is from about 1 mg/kg body weight to about 1,000 mg/kg body weight, from about 10 mg/kg body weight to about 800 mg/kg body weight, from about 20 mg/kg body weight to about 700 mg/kg body weight, from about 30 mg/kg body weight to about 600 mg/kg body weight, from about 40 mg/kg body weight to about 500 mg/kg body weight, from about 50 mg/kg body weight to about 400 mg/kg body weight, from about 75 mg/kg body weight to about 300 mg/kg body weight, or from about 100 mg/kg body weight to about 200 mg/kg body weight. In one embodiment, the dose of Fc multimer is from about 25 mg/kg body weight to about 1,000 mg/kg body weight, from about 25 mg/kg body weight to about 800 mg/kg body weight, from about 25 mg/kg body weight to about 600 mg/kg body weight, from about 25 mg/kg body weight to about 500 mg/kg body weight, from about 25 mg/kg body weight to about 400 mg/kg body weight, from about 25 mg/kg body weight to about 300 mg/kg body weight, from about 25 mg/kg body weight to about 200 mg/kg body weight, or from about 25 mg/kg body weight to about 100 mg/kg body weight.

In a separate embodiment, the pharmaceutical composition(s) of Fc multimer is administered alone or in conjunction with other therapeutic agents. In one embodiment, these agents are incorporated as part of the same pharmaceutical. In one embodiment, the Fc multimer is administered in conjunction with an immunosuppressant therapy, such as a steroid. In another embodiment, the Fc multimer is administered with any B cell or T cell modulating agent or immunomodulator.

The administration frequency of the Fc multimer depends on many factors such as the formulation, dosage, and mode of administration. In one embodiment, a dose of Fc multimer is administered multiple times every day, once every day, once every other day, once every third day, twice per week, once per week, once every two weeks, once every three weeks, or once per month.

### Therapeutic effects

The term "therapeutic effects," as used herein, describes treating the disease or disorder by improving parameters that characterize it, or, alternatively, preventing those disease/disorder parameters altogether. For example, therapeutic effects can be determined (1) *in vitro* in cell culture models of neuromyelitis optica, (2) ex *vivo* in spinal cord slice models of neuromyelitis optica, or (3) *in vivo* in rat models of disease by administering a dose of an Fc multimer. A dose of Fc multimer can be 10 to 1000 mg/kg, for example, 200 mg/kg. The Fc multimer can be administered by intravenous or non-intravenous injection or intravenous infusion. Clinical assessments of animals can be made at predetermined times until a final time point after administration of the Fc multimer. Clinical assessments can include scoring based on clinical manifestations of the specific disease or disorder. Biological samples can also be taken from the animals at predetermined times until a final time point after administration of the Fc multimer. The term "biological samples," as used herein, refers to, for example, tissue, blood, and urine. The biological samples can then be assessed for improvements in markers or indicators of neuromyelitis optica.

The term "induce," as used herein, is defined as to cause, produce, effect, create, give rise to, lead to, or promote.

In a preferred embodiment, a therapeutic effect of the Fc multimer can be indicated by an improvement in the reduction of complement-dependent cytotoxicity or antibody-dependent cytotoxicity in Chinese hamster ovary cells pre-incubated with AQP4-IgG or serum from a seropositive neuromyelitis optica patient relative to effects observed following treatment with IVIG or Fc monomers. In certain embodiments, the Fc multimer is associated with accelerated reduction of cytotoxicity in the presence of 1% or 0.5% human complement. In other embodiments, the Fc multimer is associated with accelerated reduction of cytotoxicity at a concentration of 50 µg/ml or 100 µg/ml.

In a separate preferred embodiment, a therapeutic effect of the Fc multimer can be indicated by a reduction in cytotoxicity and pathology observed in ex *vivo* slice models of rat spinal cord. Spinal cords can be incubated with AQP4-IgG and human complement to produce a neuromyelitis slice model and then immunostained with markers for astrocyte injury, such as AQP4 and GFAP, markers for demyelination, such as MBP, markers for inflammation, such as Iba1, and markers for the deposition of the complement terminal membrane attach complex, such as C5b-9. Pathology can be assessed and scored as follows: 0 - intact slice with normal AQP4, GFAP, and MBP staining; 1 - mild astrocyte injury, demyelination, inflammation, and deposition of the complement terminal membrane attack complex, as demonstrated by reduced AQP4 or GFAP staining, reduced MBP staining, increased Iba1 staining, and increased C5b-9 staining; 2 - at least one lesion with reduced AQP4 or GFAP staining, reduced MBP staining, increased Iba1 staining, and increased C5b-9 staining; 3 - multiple lesions affecting <30 % of slice area; 4 - lesions affecting 80% of slice area. Scores for each slice can be summed for a total clinical score. The therapeutic effect of an Fc multimer can be compared to treatment with no hexamer.

In another preferred embodiment, a therapeutic effect of the Fc multimer can be indicated in an experimental rat model of neuromyelitis optica induced by intracerebral injection of AQP4-IgG. The therapeutic effect can be assessed by administering doses of 3.125, 6.25, 12.5, 25, or 50 mg/kg intravenously and collecting blood at 2 hours post-administration. Serum can be collected from the rat blood and cytotoxicity assessed by incubation with AQP4-expressing Chinese hamster ovary cells pre-incubated with AQP4-IgG. In one embodiment, cytotoxicity can be assessed following a 50 mg/kg dose administration of Fc multimer and subsequent collection of blood for *in vitro* testing at various time points post-administration. In one embodiment, brains from neuromyelitis optica rats can be harvested, sectioned, and immunostained with various markers to assess pathology, including markers for astrocyte injury, such as AQP4 and GFAP, markers for demyelination, such as MBP, markers for inflammation, such as Iba1 and CD45, and markers for the deposition of the complement terminal membrane attach complex, such as C5b-9. For comparison, the non-injected contralateral hemisphere can also be stained.

### Activation of the classical complement pathway

The classical complement pathway mediates the specific antibody response and is mediated by a cascade of complement components. The cascade is mainly activated by antigen-antibody complexes. The initial component of the pathway is the protein complex C1, which is comprised of one C1q and two subunits of C1r2s2. Binding of an immunoglobulin to C1q effects the first step of activation of the classical complement pathway through activation of C1r2s2 into catalytically active subunits. The activated C1s cleaves C4 into C4a and C4b and C2 into C2a and C2b. C2a then binds C4b to form C4b2a, which is also known as C3 convertase. C3 convertase catalyzes the cleavage of C3 into C3a and C3b. C3b can then bind to activated C4b2a to form C4b2a3b, which is also known as C5 convertase. C5 convertase converts C5 to fragments C5a and C5b. C5b, together with the C6, C7, C8, and C9 components, forms a complex known as the C5b-9 complex. This complex is also known as the membrane attack complex (MAC) or terminal complement complex (TCC) and forms transmembrane channels in target cells, leading to cell lysis.

"Activation of the complete classical complement pathway", as used herein, is defined as the activation of every step of the entire classical complement pathway as described above. Activation of the complete classical complement pathway can be determined by investigating binding of the Fc multimer to C1q, the first step in activation of the classical complement pathway, and formation of C4a, C5a or soluble or membrane bound C5b-9 complex, the final effector in the classical complement pathway. For example, an Fc multimer does not induce complete activation of the classical complement pathway if the protein binds C1q but soluble C5b-9 is essentially not formed, i.e. only less than 50% of the respective positive control is formed, preferably less than 40%, preferably less than 30%, preferably less than 20%, preferably less than 10%, more preferably less than 5%. Activation of the classical complement pathway can also be determined by assessing the generation of C4a, cleavage of C2, or formation of C3 convertase. For example, an Fc multimer does not induce activation of the complete classical complement pathway if it induces the generation of C4a but either does not induce cleavage of C2 or does not induce formation of C3 convertase. "Not induce" means less than 50%, preferably less than 40%, preferably less than 30%, preferably less than 20%, preferably less than 10%, more preferably less than 5% of the respective positive control is formed.

The ability of an Fc multimer to bind human IgG and AQP4 can be determined by an *in vitro* binding assay, such as an enzyme-linked immunosorbent assay (ELISA). For example, wells of a 96-well plate can be pre-coated with human AQP4-IgG followed by the addition of Fc multimers. Purified peroxidase-labeled anti-human IgG conjugate can be added and bound conjugate can be visualized by using a color-producing peroxidase substrate, such as 3,3',5,5' tetramethylbenzidine (TMB). In one embodiment, Chinese hamster ovary cells can be incubated for one hour with AQP4-IgG or control IgG in the presence of Fc multimer. Cells can then be incubated with an anti-AQP4 antibody and, subsequently, Alexa Fluor antibodies in order to quantify fluorescence.

The ability of an Fc multimer to bind C1q can be determined by an *in vitro* binding assay, such as an enzyme-linked immunosorbent assay (ELISA). For example, wells of a 96-well plate can be pre-coated with human C1q followed by the addition of Fc multimers. Purified peroxidase-labeled anti-human IgG conjugate can be added and bound conjugate can be visualized by using a color-producing peroxidase substrate, such as 3,3',5,5' tetramethylbenzidine (TMB). In one embodiment, recombinant human C1q can be pre-incubated for one hour with Fc multimer and then added to AQP4-lgG-coated cells for one hour. C1q can be stained with a FITC-conjugated anti-C1q antibody.

Activation of the classical complement pathway by an Fc multimer can be determined by *in vitro* assays and indicated by generation of C4a and soluble C5b-9. For example, different concentrations of an Fc multimer can be incubated in whole blood or serum for a predetermined period of time and any resulting generation of C4a or soluble C5b-9 (sC5b-9) can be determined by immunodetection, such as ELISA. Concentrations of Fc multimer used may be 0.01 mg/ml to 2 mg/ml, for example, 0.04 mg/ml, 0.2 mg/ml, or 1.0 mg/ml.

Generation of C4a and sC5b-9 induced by an Fc multimer can be compared relative to the generation of these components induced by heat-aggregated gamma globulin (HAGG), a potent activator of the classical complement pathway. For example, this assay can be performed in whole blood. According to some embodiments, as described in WO 2017/129737, the Fc multimer induces less than 50% sC5b-9 generation, less than 40% sC5b-9 generation, less than 30% sC5b-9 generation, less than 20% sC5b-9 generation, or less than 10% sC5b-9 generation as compared to sC5b-9 generation induced by HAGG. In one embodiment, the Fc multimer induces less than 20% sC5b-9 generation in whole blood as compared to sC5b-9 generation induced by HAGG in whole blood. In another embodiment, the Fc multimer induces less than 10% sC5b-9 generation in whole blood as compared to sC5b-9 generation induced by HAGG in whole blood. In yet another embodiment, the Fc multimer induces no sC5b-9 generation.

The term "normal human serum activated with heat aggregated IgG" as used herein refers to a normal human serum sample where cleavage of nearly all C4 has been induced with heat aggregated IgG.

Activation of the classical complement pathway by an Fc multimer can also be determined by detecting C2 protein. If C2 protein is cleaved to C2a and C2b, the level of C2 protein decreases, indicating activation of the classical complement pathway. Different concentrations of an Fc multimer can be incubated in whole blood or serum for a predetermined period of time, for example 2 h, following which C2 protein levels can be determined by immunodetection, such as immunoblotting. Activation of the classical complement pathway is indicated by cleavage of the C2 protein. The level of C2 protein in normal human serum can be compared to the level of C2 protein resulting after preincubation with an Fc multimer to determine the amount of C2 cleavage, and therefore activation of the classical complement pathway. A known activator of the classical complement pathway, such as HAGG, can be used as a positive control for inducing cleavage of the majority of the C2 protein in normal human serum. The term "majority," as used herein, is defined as comprising greater than 50%, greater than 60%, greater than 70%, greater than 80%, or greater than 90%. In some embodiments, as described in WO 2017/129737, the Fc multimer does not induce the cleavage of the majority of C2 protein.

Activation of the classical complement pathway by an Fc multimer can also be determined by assessing formation of C3 convertase. As described above, C3 convertase consists of the C2a and C4b subunits (C4b2a). If C2 protein is not cleaved to C2a and C2b, C3 convertase cannot be formed. As such, C3 convertase formation can be assessed as described above for determining C2 protein cleavage. In some embodiments, as described in WO 2017/129737, the Fc multimer does not induce formation of C3 convertase.

### Inhibition of the classical complement pathway

Inhibition of the classical complement pathway by an Fc multimer can be determined by determining inhibition of C5a and sC5b-9 generation or by determining inhibition of cleavage of C2 protein. Different concentrations of the Fc multimer can be incubated in whole blood or serum with a known activator of the classical complement pathway. The level of sC5b-9 generated in the presence of an Fc multimer and a known activator of the classical complement pathway can then be compared to the level of sC5b-9 generated with the known activator of the classical complement pathway alone. The level of sC5b-9 generated can be determined as described above. The concentrations of Fc multimer used may be 0.01 mg/ml to 2 mg/ml, for example, 0.04 mg/ml, 0.2 mg/ml, or 1.0 mg/ml. The known activator of the classical complement pathway may be HAGG. The lower the level of sC5b-9 generated in the presence of an Fc multimer and an activator of the classical complement pathway is in comparison to the level of sC5b-9 generated in the presence of an activator of the classical complement pathway alone, the greater is the inhibition of sC5b-9 generation by the Fc multimer. In some embodiments, the Fc multimer inhibits greater than 50% sC5b-9 generation, greater than 60% sC5b-9 generation, greater than 70% sC5b-9 generation, greater than 80% sC5b-9 generation or greater than 90% sC5b-9 generation as compared to sC5b-9 generation induced by HAGG. In one embodiment, as described in WO 2017/129737, the Fc multimer inhibits greater than 80% of sC5b-9 generation induced by HAGG.

The term "inhibit," as used herein, is defined as to suppress, restrict, prevent, interfere with, stop, or block.

Inhibition of cleavage of C2 protein can be similarly determined. Different concentrations of the Fc multimer can be incubated in whole blood or serum with a known activator of the classical complement pathway. The greater the level of C2 protein in the presence of an Fc multimer and a known activator of the classical complement pathway compared to the level of C2 protein in the presence of the known activator of the classical complement pathway alone, the greater is the inhibition of C2 cleavage by the Fc multimer. The level of C2 protein can be determined as described above. The concentrations of Fc multimer used may be 0.01 mg/ml to 2 mg/ml, for example, 0.04 mg/ml, 0.2 mg/ml, or 1.0 mg/ml. The known activator of the classical complement pathway can be HAGG. In some embodiments, as described in WO 2017/129737, the Fc multimer inhibits the cleavage of the majority of C2 protein by HAGG.

Inhibition of the classical complement pathway can also be determined using a hemolysis assay for the classical complement pathway using antibody-sensitized, or opsonized, erythrocytes. For example, sheep erythrocytes, or red blood cells, can be opsonized with rabbit anti-sheep antibodies. Normal human serum (NHS) will induce lysis of opsonized erythrocytes. Fc proteins can be pre-incubated with NHS and then added to the erythrocytes and incubated for 1 h at 37 °C. The concentration of Fc construct can be from 1-1000 µg/ml, for example 2.5, 25, 50, 125, 250, or 500 µg/ml. Alternatively, Fc monomer can also be pre-incubated with NHS at the same concentrations as indicated for the Fc construct. After incubation, the mixture can be centrifuged and the degree of lysis can be determined by measuring the absorbance of released hemoglobin at 412 nm of the supernatant.

Inhibition of the classical complement pathway by the Fc multimer can be indicated by reduced lysis of erythrocytes in the mixtures that contain Fc multimer compared to the mixtures that have NHS but not Fc multimer. Inhibition of lysis of opsonized red blood cells by an Fc multimer can also be compared to lysis of opsonized red blood cells in the presence of the Fc monomer. In some embodiments, the Fc multimer inhibits lysis of opsonized sheep red blood cells as compared to Fc monomer. In one embodiment, as described in WO 2017/129737, the Fc multimer inhibits lysis of opsonized sheep red blood cells by over 70% as compared to Fc monomer.

In a preferred embodiment of the present invention, the Fc multimer prevents the pathogenesis of neuromyelitis optica by inhibiting activation of the classical complement pathway but not the alternative complement pathway.

### EXAMPLES

### Example 1: Preparation of IgG1 Fc multimers

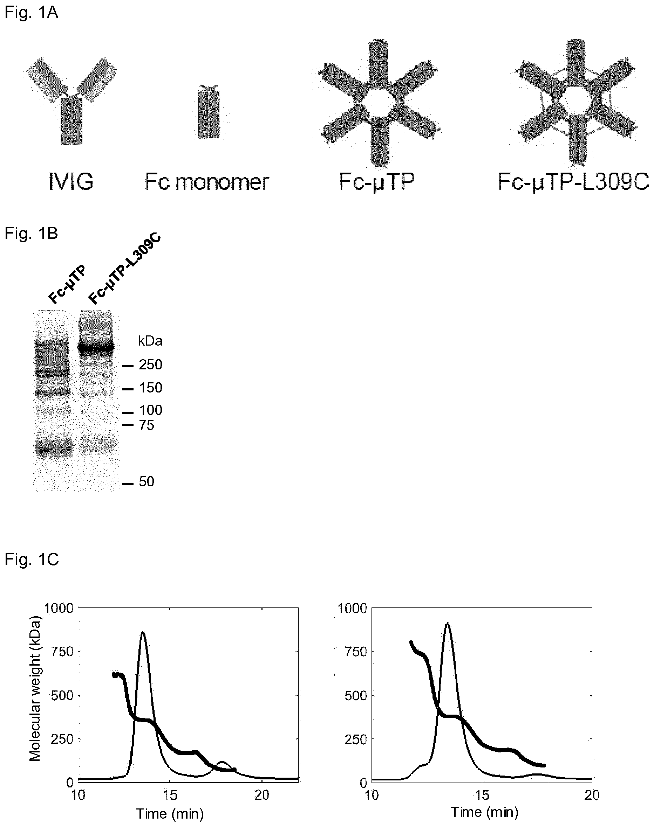

Fc-µTP (Fig 1A, left diagram) was generated by fusing the 18 amino acid residues (PTLYNVSLVMSDTAGTCY SEQ ID NO: 11) of human IgM tail piece to the C-terminus of the constant region of human IgG1 Fc fragment (amino acid residues 216-447, EU numbering; UniProtKB - P01857). Fc-µTP-L309C (Fig 1A, right diagram) was generated by mutating the Leu residue at 309 (EU numbering) of Fc-µTP to Cys. The DNA fragments encoding Fc-µTP and Fc-µTP-L309C were synthesized and codon-optimized for human cell expression by ThermoFisher Scientific (MA, USA). The DNA fragments were cloned into ApaLl and Xbal sites of pRhG4 mammalian cell expression vector using InTag positive selection method (Chen, CG et al, (2014). Nucleic Acids Res 42(4):e26; Jostock T, et al (2004). J. Immunol. Methods. 289:65-80). Briefly, Fc-µTP and Fc-µTP-L309C fragments were isolated by ApaLl and Ascl digestion. A CmR InTag adaptor comprising of BGH polyA addition sites (BGHpA) and chloramphenicol resistance gene (CmR) was also isolated by Ascl and Spel digestion (Chen, CG et al, (2014). Nucleic Acids Res 42(4):e26). The Fc molecules and the CmR InTag adaptor were co-cloned into ApaLl and Xbal sites of pRhG4 vector using T4 DNA ligase. Positive clones were selected on agar plates containing 34 µg/ml chloramphenicol. Miniprep plasmid DNA was purified using the QIAprep Spin Miniprep kit (QIAGEN, Hilden, Germany) and sequence confirmed by DNA sequencing analysis. The restriction enzymes and T4 DNA ligases were purchased from New England BioLabs (MA, USA).

The transient transfection using Expi293^{™} Expression System (Life Technologies, NY, USA) was performed according to the manufacturer's instruction. Briefly, plasmid DNA (0.8 µg) was diluted in 0.4 ml Opti-MEM and mixed gently. Expifectamine 293 Reagent (21.6 µL) was diluted in 0.4 ml Opti-MEM, mixed gently and incubated for 5 min at room temperature. The diluted Expifectamine was then added to the diluted DNA, mixed gently and incubated at room temperature for 20-30 min to allow the DNA-Expifectamine complexes to form. The DNA-Expifectamine complex was then added to the 50 ml Bioreactor tube containing 6.8 ml of Expi293 cells (2× 10⁷ cells). The cells were incubated in a 37 °C incubator with 8% CO₂ shaking at 250 rpm for approximately 16-18 h. A master mix consisting of 40 µl Enhancer 1 (Life Technologies, NY, USA), 400 µl Enhancer 2 (Life Technologies, NY, USA) and 200 µl of LucraTone^{™} Lupin was prepared and added to each Bioreactor tube. The cells were incubated for further 4 days in a 37 °C incubator with 8% CO₂ shaking at 250 rpm. Protein was harvested from supernatant centrifugation at 4000 rpm for 20 min and filtered into a clean tube using a 0.22 µm filter before HPLC quantitation and purification.

In order to produce IgG1 Fc multimers, the N-terminus of recombinant human IgG1 Fc was fused to the 18 amino acid tailpiece of IgM. The IgM tailpiece (µTP) promotes formation of pentamers and hexamers. The Fc fusion proteins were produced with either wild-type (WT) human IgG1 Fc peptide (Fc-µTP) or a variant thereof with a point mutation of leucine to cysteine at residue 309 (Fc-µTP-L309C). The leucine 309 to cysteine point mutation (Fc-µTP-L309C) was expected to provide a more stable structure than the WT (Fc-µTP) due to the formation of covalent bonds between Fc molecules.

The Fc-µTP and Fc-µTP-L309C fusion monomeric subunits result from two peptides comprising the following regions (residue numbers refer to those in SEQ ID NOs: 2 and 4, respectively):

| | |
|---|---|
| Signal peptide | residues 1-19 |
| Hinge region of human IgG1 | residues 20-34 |
| Fc region of human IgG1 | residues 35-251 |
| Tailpiece of human IgM | residues 252-269 |

The amino acid sequences for the mature forms of the Fc- µTP and Fc-µTP-L309C peptides are provided as SEQ ID NO:1 and SEQ ID NO:3, respectively. The nucleic acid coding sequences are provided as SEQ ID NO: 97 (corresponding to SEQ ID NO:9 of WO 2017129737) and SEQ ID NO: 98 (corresponding to SEQ ID NO: 10 of WO 2017129737), respectively.

During expression, the signal peptide is cleaved off to form the mature Fc-µTP and Fc-µTP-L309C fusion peptides. The sequences of the immature fusion peptides are provided in SEQ ID NOs: 2 and 4, respectively.

SDS-PAGE of the multimeric Fc proteins showed a laddering pattern for each preparation, corresponding to monomer, dimer, trimer, tetramer, pentamer and hexamers of the Fc construct. Fc-µTP-L309C, but not Fc-µTP, had a predominant band at the expected hexamer position, which was consistent with a more stable structure under the disruptive electrophoresis buffer conditions (Fig. 1B). Diagrams of the expected structures for the Fc-µTP and Fc-µTP-L309C hexamers are shown in Fig. 1A. Higher order structures, most likely dimers of hexamer, were also evident for Fc-µTP-L309C.

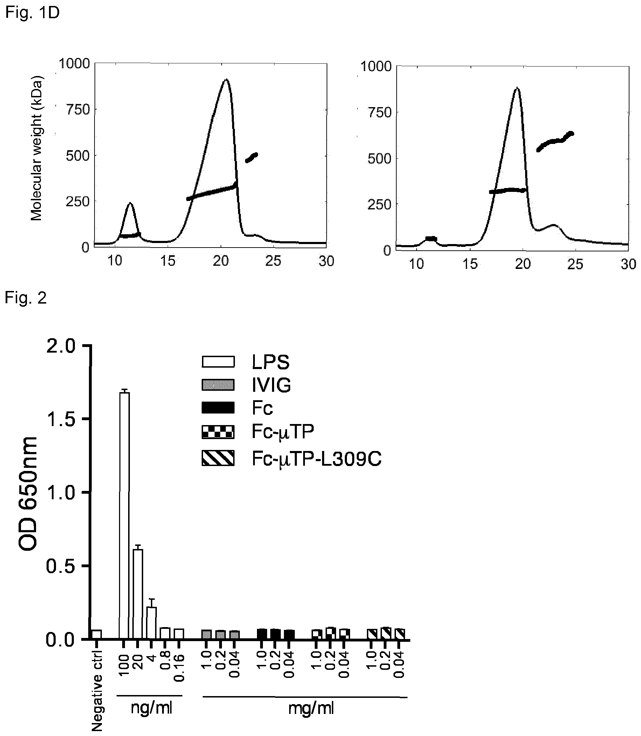

Multimerization of Fc-µTP-L309C and Fc-µTP was also examined with size exclusion chromatography (SEC) (Fig. 1C) and asymmetric flow field-flow fractionation (A4F) (Fig. 1D) of the Fc multimer preparations, followed by U.V. absorbance measurement at 280 nm (A280, thin chromatogram) and multi-angle light scattering (MALS, bold line). Similar distribution patterns with a predominant hexamer peak (approximately 85% material) were observed for each of the Fc multimer preparations with each procedure (Table 1). This is in contrast to the distinct profiles by SDS-PAGE (Fig. 1B) suggesting the presence of noncovalent hexamers in the Fc-µTP preparation. The remaining material was mostly lower order (monomer, dimer, trimer) for Fc-µTP and higher order (dimers of hexamer) for Fc-µTP-L309C.

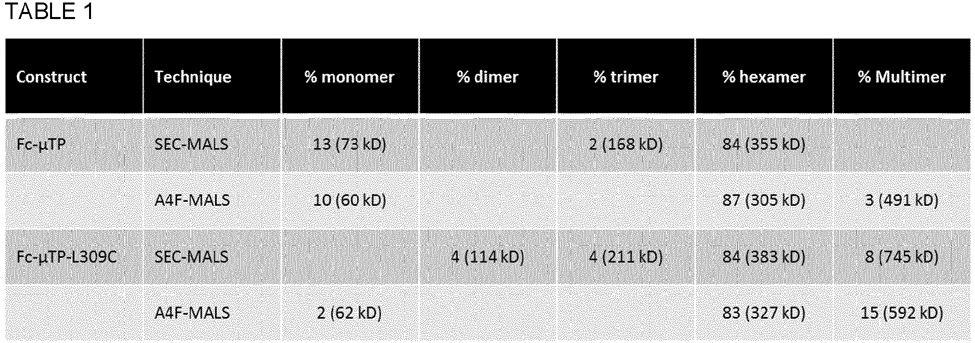

Recombinant human IgG1 Fc monomer (residues 1 to 232 of SEQ ID NO:1) was also produced and used as a control.

The Fc proteins (Fc, Fc-µTP and Fc-µTP-L309C) were considered to be endotoxin-free based on their inability to stimulate NF-κB activation in THP1 cells (Fig. 2). The human monocytic cell line, THP1, was cultured in Roswell Park Memorial Institute (RPMI) 1640 medium containing 10% fetal calf serum (FCS), 1% (100 U/ml) penicillin/streptomycin. Cell culture medium was replaced approximately every 3 days. THP1XBlue cells were derived by stable transfection of THP1 cells with a reporter plasmid expressing a secreted embryonic alkaline phosphatase (SEAP) gene under the control of a promoter inducible by the transcription factor NF-κB. Upon stimulation, THP1XBlue cells activate NF-κB and subsequently the secretion of SEAP which is readily detectable using QUANTI-blue, as medium turns purple/blue in its presence. THP1XBlue cells express all TLRs, as determined by PCR, but respond only to TLR2, TLR2/1, TLR2/6, TLR4, TLR5 and TLR8. THP1XBlue cells are resistant to the selection marker Zeocin. Cells were cultured in RPMI 1640 medium containing 10% FCS, 0.5 % (100 U/ml) penicillin/streptomycin, 100 µg/ml Normocin (Invivogen, San Diego, CA) and 200 µg/ml Zeocin (Invivogen). Cell culture medium was replaced approximately every 3 days. Lipopolysaccharide (LPS) was used as a positive control for NF-κB activation.

### Example 2: Fc hexamers inhibit complement-dependent cytotoxicity and antibody-dependent cellular cytotoxicity in AQP4-expressing cell cultures

Chinese hamster ovary (CHO) cells stably expressing human AQP4-M23 (named CHO-AQP4 cells), as described (Crane et al., 2011, J. Biol. Chem. 286, 16516-16524), were cultured at 37 °C in 5% CO₂ 95% air in F-12 Ham's Nutrient Mixture medium supplemented with 10% fetal bovine serum, 200 µg/ml geneticin, 100 U/ml penicillin and 100 µg/ml streptomycin. Human natural killer cells (NK cells) expressing the high-affinity 176V variant of the Fcy receptor, as described (Yusa et al., 2002, J. Immunol. 168, 5047-5057), were obtained from Fox Chase Cancer Center (Philadelphia, PA).

CHO-AQP4 cells were grown in 96-well plates until confluence with 25,000 cells per well. Cells were pre-incubated with 10 µg/ml AQP4-IgG (rAb-53) or neuromyelitis optica (NMO) serum (1:50) for 1 h at 23 °C. For assay of CDC, human or rat complement was pre-incubated for 1 h at 4 °C with specified concentrations of Fc preparations and then added to the AQP4-lgG-coated CHO-AQP4 cells for an additional 1 h at 23 °C. For analysis of kinetics, human complement was pre-incubated with Fc-µTP-L309C for specified times prior to addition to the AQP4-lgG-coated CHO-AQP4 cells. For assay of ADCC, CHO-AQP4 cells were incubated for 2 h at 37 °C with 5 µg/ml AQP4-IgG, without or with Fc preparations, and NK cells at an effector:target cell ratio of 4:1. CHO-AQP4 cells were then washed extensively in PBS and cell viability was measured by addition of 20% Alamar Blue (Invitrogen, Carlsbad, CA) for 45 min at 37 °C and percentage cytotoxicity determined as described (Phuan et al., 2013, Acta Neuropathol. 125, 829-840; Ratelade et al., 2014, Exp. Neurol. 225, 145-153).

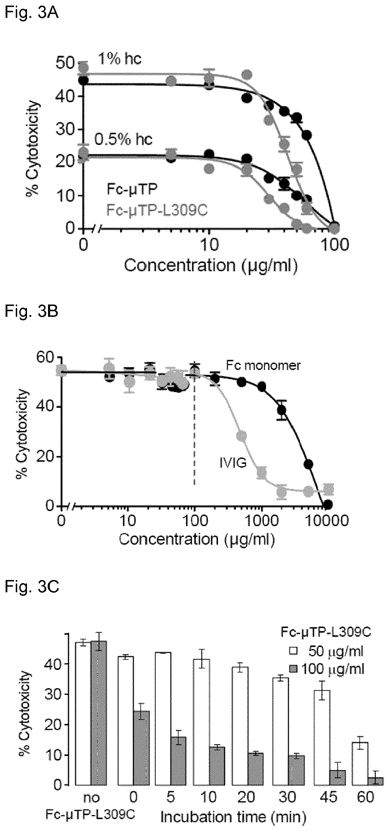

For these studies the Fc preparations were incubated with human complement (human serum) prior to addition to AQP4-IgG pre-incubated cells. Fc-µTP and Fc-µTP-L309C blocked cytotoxicity in a concentration-dependent manner with >500-fold greater potency than IVIG and >3000-fold greater potency than Fc monomers (Figs. 3A and 3B).

Kinetics studies showed rapid inhibition of CDC at a Fc-µTP-L309C concentration above its IC₅₀, though much slower inhibition at lower Fc-µTP-L309C concentration (Fig. 3C), which is consistent with a cooperative binding mechanism involving multivalent interaction of Fc-µTP-L309C with C1q.

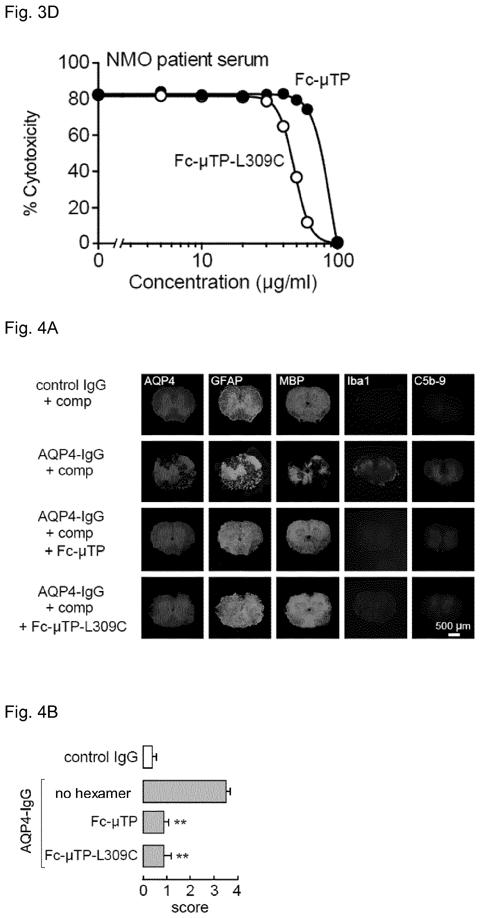

Fig. 3D shows inhibition of CDC by Fc-µTP and Fc-µTP-L309C when cytotoxicity was initiated by serum from a seropositive NMO patient rather than recombinant AQP4-IgG. Apparent IC₅₀ values were similar to those in Fig. 3A, supporting the conclusion that the Fc hexamers act on complement rather than AQP4-IgG or its binding to AQP4.

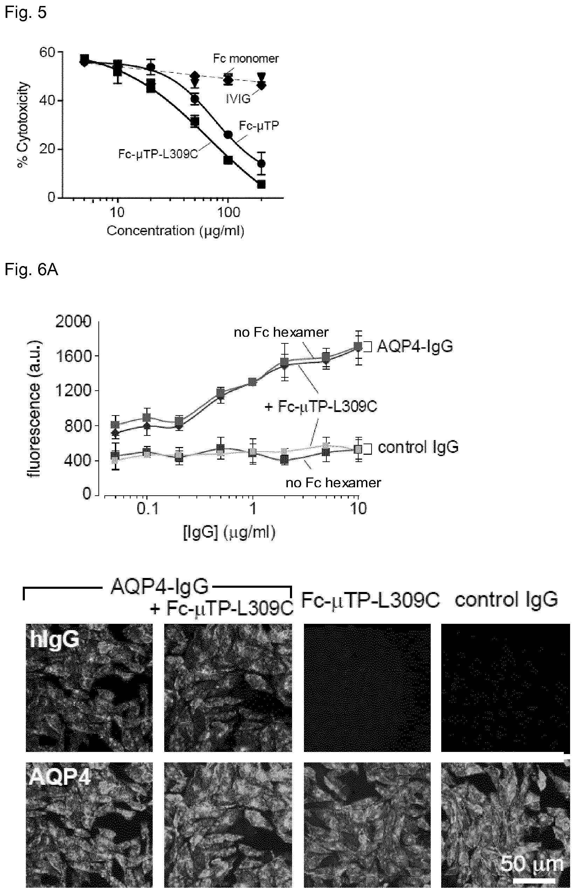

The Fc preparations were also tested for their efficacy in inhibition of ADCC produced by incubation of AQP4-expressing CHO cells with AQP4-IgG and NK cells (Phuan et al., 2013, Acta Neuropathol. 125, 829-840; Ratelade et al., 2014, Exp. Neurol. 225, 145-153; Tradtrantip et al., 2012, Ann. Neurol. 71, 314-322). ADCC was inhibited in a concentration-dependent manner by Fc-µTP and Fc-µTP-L309C with IC₅₀ ~80 µg/ml, and 50 µg/ml, respectively, with little inhibition seen for IVIG or Fc monomers in the concentration range tested (Fig. 5).

### Example 3: Fc hexamers prevent pathology in a spinal cord slice model of neuromyelitis optica (NMO)

Spinal cords were obtained from 7-day old rats and cut at 300-µm thickness using a vibratome, as described previously for mice (Zhang et al., 2011, Ann. Neurol. 70, 943-954). Transverse slices were placed on transparent membrane inserts (Millipore, Millicell-CM 0.4 µm pores, 30 mm diameter) in 6-well plates containing 1 ml culture medium, with a thin film of culture medium covering the slices. Slices were cultured in 5 % CO2 at 37 °C for 7 days in 50 % MEM, 25 % HBSS, 25 % horse serum, 1 % penicillin-streptomycin, 0.65 % glucose and 25 mM HEPES. The 7-day old slices were incubated with AQP4-IgG (5 µg/ml) and human complement (5 %) without or with Fc-µTP or Fc-µTP-L309C (50 µg/ml) for 24 h. The Fc preparations were pre-incubated with human complement at room temperature for 1 h prior to addition to cells. Spinal cords were immunostained for AQP4, GFAP, MBP, Iba1 and C5b-9, and photographed as described (Zhang et al., 2011, Ann. Neurol. 70, 943-954) and scored: 0, intact slice with normal GFAP and AQP4 staining; 1, mild astrocyte swelling and or reduced AQP4 staining; 2, at least one lesion with loss of GFAP and AQP4 staining; 3, multiple lesions affecting >30 % of slice area; 4, lesions affecting >80 % of slice area (Phuan et al., 2013, Acta Neuropathol. 125, 829-840; Ratelade et al., 2014, Exp. Neurol. 225, 145-153; Zhang et al., 2011, Ann. Neurol. 70, 943-954).

Data are presented as mean ± S.E.M. Statistical comparisons were made using the non-parametric Mann-Whitney test when comparing two groups.

CDC inhibition studies were also done in an ex vivo spinal cord slice model of NMO, in which 7-day cultured rat spinal cord slices show astrocyte injury (loss of AQP4 and GFAP), demyelination (reduced MPB staining), inflammation (increased Iba-1 staining) and deposition of the complement terminal membrane attack complex (C5b-9) following 24 h incubation with AQP4-IgG and human complement (Phuan et al., 2013, Acta Neuropathol. 125, 829-840; Zhang et al., 2011, Ann. Neurol. 70, 943-954). Immunofluorescence of AQP4-IgG/complement-treated spinal cord slices showed the expected pathological changes, which were largely prevented by Fc-µTP and Fc-µTP-L309C (Fig. 4A). Fig. 4B summarizes pathology scores.

### Example 4: Fc multimers inhibit hemolysis by the classical complement pathway

To investigate Fc protein effects on the classical pathway, sheep erythrocytes (Siemens) were sensitized with rabbit anti-sheep antibodies (Ambozeptor 6000; Siemens) and diluted to 4×10⁸ cells/mL GVB²⁺ (GVB, 0.15 mM CaCl₂, 0.5 mM MgCl₂). To assess inhibition of hemolysis by Fc-µTP-L309C, the recombinant protein was pre-incubated in 1% or 5% human complement for 30 min at room temperature and subsequently added to the erythrocytes at a 1/1 (v/v) ratio and incubated during 1 h at 37 °C in a microtiter-plate shaking device. The concentrations of Fc-µTP-L309C tested ranged from 0.1 to 5µg/ml. After adding ice-cold GVBE (GVB, 10 mM EDTA) and centrifugation (5 min at 1250 × g, 4 °C), hemolysis was determined in the supernatant by measuring the absorbance of released hemoglobin at 412 nm.

To investigate Fc protein effects on the alternative pathway, rabbit erythrocytes (Jackson Laboratories) were washed and diluted to 2×10⁸ cells/mL GVB/MgEGTA (GVB, 5 mM MgEGTA). To assess inhibition of hemolysis by Fc-µTP-L309C, the recombinant protein was pre-incubated in 5% or 10% human complement for 30 min at room temperature and subsequently added to the erythrocytes at a 2/1 (v/v) ratio and incubated during 1 h at 37 °C in a microtiter-plate shaking device. The concentrations of Fc-µTP-L309C tested ranged from 0.1 to 5µg/ml. After adding ice-cold GVBE and centrifugation (10 min at 1250 × g), hemolysis was determined in the supernatant by measuring the absorbance of released hemoglobin at 412 nm.

Fc-µTP-L309C greatly inhibited hemolysis of the classical complement pathway at both 1% and 5% concentrations of human complement (Fig. 6C(ii)). Fc-µTP-L309C did not inhibit the alternative complement pathway in rabbit red blood cells (Fig. 6C(iii)).

### Example 5: Fc multimers regulate the pathogenesis of neuromyelitis optica by preventing binding of C1q to bound AQP4-IgG

CHO-AQP4 cells were grown on 96-well plates for 24 h. After blocking with 1% BSA in PBS, cells were incubated with AQP4-IgG or control IgG without or with 100 mg/ml Fc-µTP-L309C at 23 °C for 1 h. Cells were then washed with PBS and incubated with Alexa Fluor 594-goat anti-human IgG secondary antibody, F(ab')₂-fragment specific (1:500; Jackson ImmunoResearch, West Grove, PA) for 1 h. Cells were then rinsed three times with PBS and fluorescence quantified using a plate reader at excitation/emission wavelengths of 591/614 nm. For human IgG and AQP4 immunostaining, cells were incubated for 1 h at 23 °C with 10 µg/ml AQP4-IgG or control IgG in the absence or presence of 100 µg/ml Fc-µTP-L309C. Cells were then fixed in 4% PFA for 15 min and permeabilized with 0.1% Triton X-100. After blocking with 1% BSA, cells were incubated for 1 h with 0.4 µg/ml polyclonal, AQP4 C-terminal-specific rabbit anti-AQP4 antibody (Santa Cruz Biotechnology, Dallas, TX). Cells were rinsed with PBS and incubated for 1 h with Alexa Fluor 594- the F(ab')₂ fragment-specific antibody (1:400) and Alexa Fluor-488 goat anti-rabbit IgG secondary antibody (1:400; Invitrogen). To assay C1q binding, CHO-AQP4 cells were pre-incubated with 20 µg/ml AQP4-IgG for 1 h at 23 °C, and then washed with PBS. Recombinant human C1q (60 µg/ml) was pre-incubated for 1 h with Fc monomers or Fc-µTP-L309C and then added to AQP4-lgG-coated cells for 1 h. Cells were washed, fixed and C1q was stained with a rabbit FITC-conjugated anti-C1q antibody (1:50; Abcam, Cambridge, MA).

Neuromyelitis optica (NMO) pathogenesis is initiated by AQP4-IgG binding to membrane-bound AQP4, followed by binding of the initial complement protein C1q to the Fc region of bound AQP4-IgG. Fig. 6A shows that Fc-µTP-L309C at 100 µg/ml did not inhibit AQP4-IgG binding to AQP4 on CHO cells, as assayed using a fluorescent secondary antibody that recognizes the F(ab')₂ fragment of the primary antibody. Fig. 6B shows that Fc-µTP-L309C prevented binding of purified C1q to AQP4-bound AQP4-IgG, as assayed by C1q immunofluorescence, which is consistent with one of the actions of Fc-µTP-L309C being avid binding to aqueous-phase C1q.

### Example 6: Fc-µTP-L309C prevents pathology in an experimental rat model of neuromyelitis optica (NMO)

Experiments were done using weight-matched female Sprague Dawley rats (250-300 g, age 9-12 weeks). Rats received Fc-µTP-L309C at 3.125, 6.25, 12.5, 25, 50 mg/kg intravenously and blood was collected at 2 h. The blood was left to clot at room temperature for 30 min, centrifuged at 2,000 × g for 10 min at 4 °C, and serum was collected and frozen at 20 °C overnight. Serum was used in CDC assays, as described above, in which 2% rat serum was added to 1.25-10 µg/ml AQP4-IgG for 1 h at 23 °C. In some studies rat blood was collected at specified times after intravenous injection of 50 mg/kg Fc-µTP-L309C and subject to CDC assay.

AQP4-IgG was delivered by intracerebral injection as described (Yao and Verkman, 2017, Acta Neurolpathol. Commun. 5, 15). Briefly, rats were anesthetized using ketamine (100 mg/kg) and xylazine (10 mg/kg) and then mounted onto a stereotaxic frame. Following a midline scalp incision, a burr hole of 1 mm diameter was created 0.5 mm anterior and 3.5 mm lateral of bregma. A 40-µm diameter glass needle was inserted 5 mm deep to infuse 30 or 40 µg AQP4-IgG in a total volume of 3-6 µL over 10 minutes by pressure injection. At day 5 rats were deeply anesthetized, followed by a transcardiac perfusion through the left ventricle with 200 ml of heparinized PBS and then 100 ml of 4% paraformaldehyde (PFA) in PBS. Brains were fixed in 4% PFA, left overnight at 4 °C in 30% sucrose and embedded in OCT.

Fixed brains were frozen, sectioned (10-µm thickness) and incubated in blocking solution (PBS, 1% bovine serum albumin, 0.2% Triton X-100) for 1 h prior to overnight incubation (4 °C) with primary antibodies: AQP4 (1:200, Santa Cruz Biotechnology, Santa Cruz, CA), GFAP (1:100, Millipore ), myelin basic protein (MBP) (1:200, Santa Cruz Biotechnology), ionized calcium-binding adaptor molecule-1 (Iba1; 1:1000; Wako, Richmond, VA), C5b-9 (1:50, Hycult Biotech, Uden, The Netherlands) or CD45 (1:10, BD Biosciences, San Jose, CA), followed by the appropriate fluorescent secondary antibody (1:200, Invitrogen, Carlsbad, CA). Sections were mounted with VECTASHIELD (Vector Laboratories, Burlingame, CA) for visualization on a Leica fluorescence microscope.

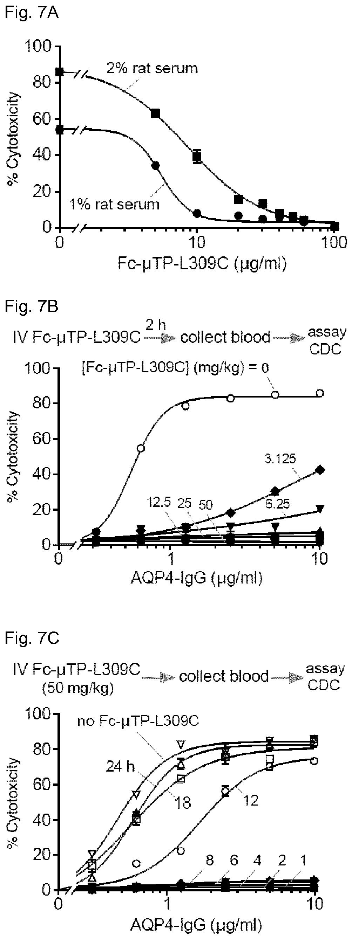

In vivo efficacy studies were done using an established experimental model of NMO in rats in which NMO pathology is created by intracerebral administration of AQP4-IgG (Asavapanumas et al., 2014, Acta Neuropathol. 127, 539-551; Yao and Verkman 2017, Acta Neurolpathol. Commun. 5, 15). The model was done in rats rather than mice because rats have human-like complement activity whereas mice have a largely inactive classical complement system (Ratelade and Verkman, 2014, Mol. Immunol. 62, 103-114). Fc-µTP-L309C was effective in inhibiting CDC produced by AQP4-IgG and rat complement (Fig. 7A), with several-fold greater potency than found with human complement in Fig. 3A.

To establish a Fc-µTP-L309C dosing regimen to give therapeutic blood levels for efficacy studies, rats were administered different amounts of Fc-µTP-L309C intravenously, and complement activity of serum taken at 2 h was assayed in vitro by measurement of CDC in AQP4-expressing CHO cells that were preincubated with the rat serum and AQP4-IgG (Fig. 7B). Cytotoxicity was prevented in sera taken at 2 h from rats administrated Fc-µTP-L309C at a dose of 12.5 mg/kg or higher. Fig. 7C shows the time course of rat serum-induced cytotoxicity following administration of a single intravenous dose of 50 mg/kg Fc-µTP-L309C. Cytotoxicity was prevented for at least 8 hours.

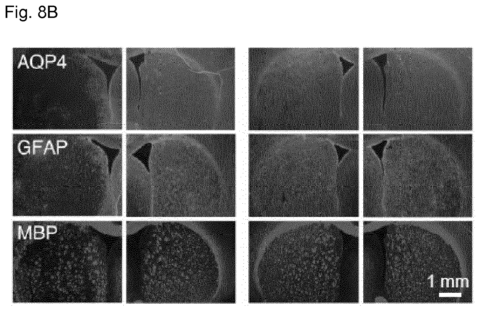

A short-term efficacy study was done in which Fc-µTP-L309C at 50 mg/kg was administered at the time of and 12 h after intracerebral injection of AQP4-IgG (Fig. 8A). Immunofluorescence of AQP4-IgG treated rats showed astrocyte injury (loss of astrocyte markers AQP4 and GFAP) in an area surrounding the administration site, as well as demyelination (reduced MBP immunofluorescence), inflammation (lba-1 and CD45) and deposition of activated complement (C5b-9) (Fig. 8A). The increased GFAP expression surrounding the lesion represents reactive gliosis. Immunofluorescence of the non-injected contralateral hemisphere is shown for comparison. Remarkably reduced pathology was seen in the Fc-pTP-L309C-treated rats, in which AQP4, GFAP, MBP, C5b-9 and CD45 immunofluorescence were similar to that in untreated rats and the contralateral hemisphere of Fc-µTP-L309C-treated rats. In a further study, a greater amount of AQP4-IgG was injected in order to produce massive NMO pathology in nearly the whole ipsilateral hemisphere (Fig. 8B). Fc-µTP-L309C fully prevented the loss of AQP4, GFAP and MBP immunofluorescence.

## Claims

1. An Fc multimeric protein for use in the treatment of neuromyelitis optica, where in the Fc multimeric protein comprises six IgG Fc fusion monomers, wherein each Fc fusion monomer comprises two Fc fusion polypeptide chains and each Fc fusion polypeptide chain comprises an IgG Fc polypeptide and an IgM tailpiece.

2. The Fc multimeric protein for use according to claim 1, wherein each Fc fusion polypeptide chain further comprises an IgG hinge region and does not comprise a Fab polypeptide.

3. The Fc multimeric protein for use according to claims 1-2, wherein the Fc fusion polypeptide chains comprise an IgG1 hinge region and an IgG1 Fc polypeptide.

4. The Fc multimeric protein for use according to claims 1-3, wherein the Fc fusion polypeptide chains are represented by SEQ ID NO: 1.

5. The Fc multimeric protein for use according to claims 1-3, wherein the Fc fusion polypeptide chains are represented by SEQ ID NO: 2.

6. The Fc multimeric protein for use according to claims 1-3, wherein the Fc fusion polypeptide chains are represented by SEQ ID NO: 3 and, at position 309 of each IgG1 Fc polypeptide, a leucine is mutated to a cysteine.

7. The Fc multimeric protein for use according to claims 1-3, wherein the Fc fusion polypeptide chains are represented by SEQ ID NO: 4 and, at position 309 of each IgG1 Fc polypeptide, a leucine is mutated to a cysteine.

8. The Fc multimeric protein for use according to claims 1-7, wherein the Fc fusion polypeptide chains have up to 5 conservative amino acid changes.

9. The Fc multimeric proteinfor use according to claims 1-8, wherein the Fc multimeric protein inhibits complement-dependent cytotoxicity and antibody-dependent cellular cytotoxicity in an *in vitro* model of NMO.

10. The Fc multimeric proteinfor use according to claims 1-9, wherein the Fc multimeric protein inhibits complement-dependent cytotoxicity and pathology ex *vivo* in a spinal cord slice model of neuromyelitis optica.

11. The Fc multimeric protein for use according to claims 1-10, wherein the Fc multimeric protein prevents the pathogenesis of neuromyelitis optica by inhibiting activation of the classical complement pathway but not the alternative complement pathway.

12. The Fc multimeric protein for use according to claims 1-11, wherein the Fc multimeric protein or the recombinant human Fc hexamer prevents cytotoxicity and pathology *in vivo* in a rat model of neuromyelitis optica.

## Patentansprüche

1. Fc-Multimer-Protein zur Verwendung bei der Behandlung von Neuromyelitis optica, wobei das Fc-Multimer-Protein sechs IgG-Fc-Fusionsmonomere umfasst, wobei die Fc-Fusionsmonomere jeweils zwei Fc-Fusionspolypeptidketten umfassen und die Fc-Fusionspolypeptidketten jeweils ein IgG-Fc-Polypeptid und ein IgM-Endstück umfassen.

2. Fc-Multimer-Protein zur Verwendung nach Anspruch 1, wobei die Fc-Fusions polypeptidketten ferner jeweils eine IgG-Hinge-Region und kein Fab-Polypeptid umfassen.

3. Fc-Multimer-Protein zur Verwendung nach Anspruch 1-2, wobei die Fc-Fusionspolypeptidketten eine IgG1-Hinge-Region und ein IgG1-Fc-Polypeptid umfassen.

4. Fc-Multimer-Protein zur Verwendung nach Anspruch 1-3, wobei die Fc-Fusionspolypeptidketten unter SEQ ID NO: 1 dargestellt sind.

5. Fc-Multimer-Protein zur Verwendung nach Anspruch 1-3, wobei die Fc-Fusionspolypeptidketten unter SEQ ID NO: 2 dargestellt sind.

6. Fc-Multimer-Protein zur Verwendung nach Anspruch 1-3, wobei die Fc-Fusionspolypeptidketten unter SEQ ID NO: 3 dargestellt sind und an Position 309 der einzelnen IgG1-Fc-Polypeptide jeweils ein Leucin zu einem Cystein mutiert ist.

7. Fc-Multimer-Protein zur Verwendung nach Anspruch 1-3, wobei die Fc-Fusionspolypeptidketten unter SEQ ID NO: 4 dargestellt sind und an Position 309 der einzelnen IgG1-Fc-Polypeptide jeweils ein Leucin zu einem Cystein mutiert ist.

8. Fc-Multimer-Protein zur Verwendung nach Anspruch 1-7, wobei die Fc-Fusionspolypeptidketten bis zu 5 konservative Aminosäureänderungen aufweisen.

9. Fc-Multimer-Protein zur Verwendung nach Anspruch 1-8, wobei das Fc-Multimer-Protein komplementabhängige Zytotoxizität und antikörperabhängige zelluläre Zytotoxizität in einem In-vitro-Modell von NMO hemmt.

10. Fc-Multimer-Protein zur Verwendung nach Anspruch 1-9, wobei das Fc-Multimer-Protein komplementabhängige Zytotoxizität und Pathologie ex *vivo* in einem Rückenmarkscheiben-Modell von Neuromyelitis optica hemmt.

11. Fc-Multimer-Protein zur Verwendung nach Anspruch 1-10, wobei das Fc-Multimer-Protein die Pathogenese von Neuromyelitis optica durch Hemmen der Aktivierung des klassischen, jedoch nicht des alternativen Komplementwegs verhindert.

12. Fc-Multimer-Protein zur Verwendung nach Anspruch 1-11, wobei das Fc-Multimer-Protein oder das rekombinante Mensch-Fc-Hexamer Zytotoxizität und Pathologie *in vivo* in einem Ratte-Modell von Neuromyelitis optica hemmt.

## Revendications

1. Protéine multimérique de Fc destinée à être utilisée dans le traitement de la neuromyélite optique, la protéine multimérique de Fc comprenant six monomères de fusion de Fc d'IgG, dans laquelle chaque monomère de fusion de Fc comprend deux chaînes polypeptidiques de fusion de Fc et chaque chaîne polypeptidique de fusion de Fc comprend un polypeptide Fc d'IgG et une partie de queue d'IgM.

2. Protéine multimérique de Fc destinée à être utilisée selon la revendication 1, dans laquelle chaque chaîne polypeptidique de fusion de Fc comprend en outre une région charnière d'IgG et ne comprend pas de polypeptide Fab.

3. Protéine multimérique de Fc destinée à être utilisée selon les revendications 1-2, dans laquelle les chaînes polypeptidiques de fusion de Fc comprennent une région charnière d'IgG1 et un polypeptide Fc d'IgG1.

4. Protéine multimérique de Fc destinée à être utilisée selon les revendications 1-3, dans laquelle les chaînes polypeptidiques de fusion de Fc sont représentées par la SEQ ID n° : 1.

5. Protéine multimérique de Fc destinée à être utilisée selon les revendications 1-3, dans laquelle les chaînes polypeptidiques de fusion de Fc sont représentées par la SEQ ID n° : 2.

6. Protéine multimérique de Fc destinée à être utilisée selon les revendications 1-3, dans laquelle les chaînes polypeptidiques de fusion de Fc sont représentées par la SEQ ID n° : 3 et, à la position 309 de chaque polypeptide Fc d'IgG1, une leucine est mutée en une cystéine.

7. Protéine multimérique de Fc destinée à être utilisée selon les revendications 1-3, dans laquelle les chaînes polypeptidiques de fusion de Fc sont représentées par la SEQ ID n° : 4 et, à la position 309 de chaque polypeptide Fc d'IgG1, une leucine est mutée en une cystéine.

8. Protéine multimérique de Fc destinée à être utilisée selon les revendications 1-7, dans laquelle les chaînes polypeptidiques de fusion de Fc ont jusqu'à 5 changements d'acides aminés conservatifs.

9. Protéine multimérique de Fc destinée à être utilisée selon les revendications 1-8, la protéine multimérique de Fc inhibant une cytotoxicité dépendante du complément et une cytotoxicité cellulaire dépendante d'anticorps dans un modèle de NMO *in vitro.*

10. Protéine multimérique de Fc destinée à être utilisée selon les revendications 1-9, la protéine multimérique de Fc inhibant ex *vivo* une cytotoxicité dépendante du complément et une pathologie dans un modèle sur lame de moelle épinière de la neuromyélite optique.

11. Protéine multimérique de Fc destinée à être utilisée selon les revendications 1-10, la protéine multimérique de Fc empêchant la pathogénie de la neuromyélite optique en inhibant l'activation de la voie classique du complément mais non la voie alternative du complément.

12. Protéine multimérique de Fc destinée à être utilisée selon les revendications 1-11, la protéine multimérique de Fc, ou l'hexamère de Fc humain recombinant, empêchant in *vivo* une cytotoxicité et une pathologie dans un modèle de rat de la neuromyélite optique.
